# EUROPEAN PATENT SPECIFICATION

(11) **EP 1 020 529 B1**
(45) Date of publication and mention of the grant of the patent: **01.06.2005**
(21) Application number: 99203878.6
(22) Date of filing: 19.11.1999
(51) Int. Cl.: C12N 15/861, C12N 15/62, C12N 15/34, C12N 5/10, C07K 14/075, A61K 48/00, A61P 9/00

(54) **Gene delivery vectors provided with a tissue tropism for smooth muscle cells, and/or endothelial cells**
Genverabreichende Vektoren mit Gewebetropismus für glatte Muskelzellen und/oder Endothelzellen
Vecteurs d'apport de gènes munis d'un tropisme pour les cellules des muscles lisses et/ou les cellules endothéliales

(30) Priority: 20.11.1998 EP 98203921
(43) Date of publication of application: 19.07.2000
(73) Proprietor: Crucell Holland B.V., 2333 CN Leiden (NL)
(72) Inventor: Havenga, Menzo Jans Emco, 2401 KG Alphen aan de Rijn (NL); Vogels, Ronald, 3461 HW Linschoten (NL); Bout, Abraham, 2751 XL Moerkapelle (NL)
(74) Representative: Klein, Bart

(56) References cited:
- WO-A-97/20051
- WO-A-98/22609
- WO-A-98/33929
- WO-A-98/44121
- GALL J ET AL: "Adenovirus type 5 and 7 capsid chimera: fiber replacement alters receptor tropism without affecting primary immune neutralization epitopes" J VIROL., vol. 70, no. 4, April 1996 (1996-04), pages 2116-2123, XP002050655
- WICKHAM T ET AL: "Targeted adenovirus gene transfer to endothelial and smooth muscle cells by using bispecific antibodies" J VIROL., vol. 70, no. 10, October 1996 (1996-10), pages 6831-6838, XP002911349
- FALLAUX F ET AL: "New helper cells and matched early region 1-deleted adenovirus vectors prevent generation of replication-competent adenoviruses" HUM GENE THER., vol. 9, 1 September 1998 (1998-09-01), pages 1909-1917, XP002111070
- ROELVINK P ET AL: "The coxsackievirus-adenovirus receptor protein can function as a cellular attachment protein for adenovirus serotypes from subgroups A, C, D, E, and F" J VIROL., vol. 72, no. 10, October 1998 (1998-10), XP002111071

## Description

### FIELD OF THE INVENTION

The invention relates to the field of molecular genetics and medicine. In particular the present invention relates to the field of gene therapy, more in particular to gene therapy using adenoviruses.

### BACKGROUND OF THE INVENTION

In gene therapy, genetic information is usually delivered to a host cell in order to either correct (supplement) a genetic deficiency in said cell, or to inhibit an undesired function in said cell, or to eliminate said host cell. Of course the genetic information can also be intended to provide the host cell with a desired function, e.g. to supply a secreted protein to treat other cells of the host, etc.

Many different methods have been developed to introduce new genetic information into cells. Although many different systems may work on cell lines cultured in vitro, only the group of viral vector mediated gene delivery methods seems to be able to meet the required efficiency of gene transfer in vivo. Thus for gene therapy purposes most of the attention is directed toward the development of suitable viral vectors. Today, most of the attention for the development of suitable viral vectors is directed toward those vectors that are based on adenoviruses. These adenovirus vectors can deliver foreign genetic information very efficiently to target cells in vivo. Moreover, obtaining large amounts of adenovirus vectors is for most types of adenovirus vectors not a problem. Adenovirus vectors are relatively easy to concentrate and purify. Moreover, studies in clinical trials have provided valuable information on the use of these vectors in patients.

There are a lot of reasons for using adenovirus vectors for the delivery of nucleic acid to target cells in gene therapy protocols. However, some characteristics of the current vectors limit their use in specific applications. For instance endothelial cells and smooth muscle cells are not easily transduced by the current generation of adenovirus vectors. For many gene therapy applications, such as applications in the cardiovascular area, preferably these types of cells should be genetically modified. On the other hand, in some applications, even the very good in vivo delivery capacity of adenovirus vectors is not sufficient and higher transfer efficiencies are required. This is the case, for instance, when most cells of a target tissue need to be transduced.

The present invention was made in the course of the manipulation of adenovirus vectors. In the following section therefore a brief introduction to adenoviruses is given.

### Adenoviruses

Adenoviruses contain a linear double-stranded DNA molecule of approximately 36000 base pairs. It contains identical Inverted Terminal Repeats (ITR) of approximately 90-140 base pairs with the exact length depending on the serotype. The viral origins of replication are within the ITRs exactly at the genome ends. The transcription units are divided in early and late regions. Shortly after infection the E1A and E1B proteins are expressed and function in transactivation of cellular and adenoviral genes. The early regions E2A and E2B encode proteins (DNA binding protein, pre-terminal protein and polymerase) required for the replication of the adenoviral genome (reviewed in van der Vliet, 1995). The early region E4 encodes several proteins with pleiotropic functions e.g. transactivation of the E2 early promoter, facilitating transport and accumulation of viral mRNAs in the late phase of infection and increasing nuclear stability of major late pre-mRNAs (reviewed in Leppard, 1997). The early region 3 encodes proteins that are involved in modulation of the immune response of the host (Wold *et al*, 1995). The late region is transcribed from one single promoter (major late promoter) and is activated at the onset of DNA replication. Complex splicing and polyadenylation mechanisms give rise to more than 12 RNA species coding for core proteins, capsid proteins (penton, hexon, fiber and associated proteins), viral protease and proteins necessary for the assembly of the capsid and shut-down of host protein translation(Imperiale, M.J., Akusjnarvi, G. and Leppard, K.N. (1995) Post-transcriptional control of adenovirus gene expression. In: The molecular repertoire of adenoviruses I. P139-171. W. Doerfler and P. Bohm (eds), Springer-Verlag Berlin Heidelberg).

### Interaction between virus and host cell

The interaction of the virus with the host cell has mainly been investigated with the serotype C viruses Ad2 and Ad5. Binding occurs via interaction of the knob region of the protruding fiber with a cellular receptor. The receptor for Ad2 and Ad5 and probably more adenoviruses is known as the Coxsackievirus and Adenovirus Receptor' or CAR protein (Bergelson *et al,* 1997). Internalization is mediated through interaction of the RGD sequence present in the penton base with cellular integrins (Wickham *et al*, 1993). This may not be true for all serotypes, for example serotype 40 and 41 do not contain a RGD sequence in their penton base sequence (Kidd et al, 1993).

### The fiber protein

The initial step for successful infection is binding of adenovirus to its target cell, a process mediated through fiber protein. The fiber protein has a trimeric structure (Stouten et al, 1992) with different lengths depending on the virus serotype (Signas et al, 1985; Kidd et al, 1993). Different serotypes have polypeptides with structurally similar N and C termini, but different middle stem regions. The first 30 amino acids at the N terminus are involved in anchoring of the fiber to the penton base (Chroboczek et al, 1995), especially the conserved FNPVYP region in the tail (Arnberg et al, 1997). The C-terminus, or knob, is responsible for initial interaction with the cellular adenovirus receptor. After this initial binding secondary binding between the capsid penton base and cell-surface integrins leads to internalization of viral particles in coated pits and endocytosis (Morgan et al, 1969; Svensson and Persson, 1984; Varga et al, 1991; Greber et al, 1993; Wickham et al, 1993). Integrins are αβ-heterodimers of which at least 14 α-subunits and 8 β-subunits have been identified (Hynes, 1992). The array of integrins expressed in cells is complex and will vary between cell types and cellular environment. Although the knob contains some conserved regions, between serotypes, knob proteins show a high degree of variability, indicating that different adenovirus receptors exist.

### Adenoviral serotypes

At present, six different subgroups of human adenoviruses have been proposed which in total encompass approximately 50 distinct adenovirus serotypes. Besides these human adenoviruses, many animal adenoviruses have been identified (see e.g. Ishibashi and Yasue, 1984). A serotype is defined on the basis of its immunological distinctiveness as determined by quantitative neutralization with animal antiserum (horse, rabbit). If neutralization shows a certain degree of cross-reaction between two viruses, distinctiveness of serotype is assumed if A) the hemagglutinins are unrelated, as shown by lack of cross-reaction on hemagglutination-inhibition, or B) substantial biophysical/biochemical differences in DNA exist (Francki et al, 1991). The serotypes identified last (42-49) were isolated for the first time from HIV infected patients (Hierholzer et al, 1988; Schnurr et al, 1993). For reasons not well understood, most of such immuno-compromised patients shed adenoviruses that were never isolated from immuno-competent individuals (Hierholzer et al, 1988, 1992; Khoo et al, 1995).

Besides differences towards the sensitivity against neutralizing antibodies of different adenovirus serotypes, adenoviruses in subgroup C such as Ad2 and Ad5 bind to different receptors as compared to adenoviruses from subgroup B such as Ad3 and Ad7 (Defer et al, 1990; Gall *et al,* 1996). Likewise, it was demonstrated that receptor specificity could be altered by exchanging the Ad3 knob protein with the Ad 5 knob protein, and vice versa (Krasnykh et al, 1996; Stevenson et al, 1995, 1997). Serotypes 2, 4,5 and 7 all have a natural affiliation towards lung epithelia and other respiratory tissues. In contrast, serotypes 40 and 41 have a natural affiliation towards the gastrointestinal tract. These serotypes differ in at least capsid proteins (penton-base, hexon), proteins responsible for cell binding (fiber protein), and proteins involved in adenovirus replication. It is unknown to what extend the capsid proteins determine the differences in tropism found between the serotypes. It may very well be that post-infection mechanisms determine cell type specificity of adenoviruses. It has been shown that adenoviruses from serotypes A (Ad12 and Ad31),C (Ad2 and Ad5),D (Ad9 and Ad15),E (Ad4) and F (Ad41) all are able to bind labeled, soluble CAR (sCAR) protein when immobilized on nitrocellulose. Furthermore, binding of adenoviruses from these serotypes to Ramos cells, that express high levels of CAR but lack integrins (Roelvink *et al*, 1996), could be efficiently blocked by addition of sCAR to viruses prior to infection (Roelvink *et al*, 1998). However, the fact that (at least some) members of these subgroups are able to bind CAR does not exclude that these viruses have different infection efficiencies in various cell types. For example subgroup D serotypes have relatively short fiber shafts compared to subgroup A and C viruses. It has been postulated that the tropism of subgroup D viruses is to a large extend determined by the penton base binding to integrins (Roelvink *et al*, 1996; Roelvink *et al,* 1998). Another example is provided by Zabner *et al,* 1998 who have tested 14 different serotypes on infection of human ciliated airway epithelia (CAE) and found that serotype 17 (subgroup D) was bound and internalized more efficiently then all other viruses, including other members of subgroup D. Similar experiments using serotypes from subgroup A-F in primary fetal rat cells showed that adenoviruses from subgroup A and B were inefficient whereas viruses from subgroup D were most efficient (Law *et al*, 1998). Also in this case viruses within one subgroup displayed different efficiencies. The importance of fiber binding for the improved infection of Ad17 in CAE was shown by Armentano *et al* (WO 98/22609) who made a recombinant LacZ Ad2 virus with a fiber gene from Ad17 and showed that the chimaeric virus infected CAE more efficient then LacZ Ad2 viruses with Ad2 fibers.

Thus despite their shared ability to bind CAR, differences in the length of the fiber, knob sequence and other capsid proteins e.g. penton base of the different serotypes may determine the efficiency by which an adenovirus infects a certain target cell. Of interest in this respect is the ability of Ad5 and Ad2 fibers but not of Ad3 fibers to bind to fibronectin III and MHC class 1 α2 derived peptides. This suggests that adenoviruses are able to use cellular receptors other than CAR (Hong *et al,* 1997). Serotypes 40 and 41 (subgroup F) are known to carry two fiber proteins differing in the length of the shaft. The long shafted 41L fiber is shown to bind CAR whereas the short shafted 41S is not capable of binding CAR (Roelvink *et al*, 1998). The receptor for the short fiber is not known.

### Adenoviral gene delivery vectors

Most adenoviral gene delivery vectors currently used in gene therapy are derived from the serotype C adenoviruses Ad2 or Ad5. The vectors have a deletion in the E1 region, where novel genetic information can be introduced. The E1 deletion renders the recombinant virus replication defective. It has been demonstrated extensively that recombinant adenovirus, in particular serotype 5 is suitable for efficient transfer of genes *in vivo* to the liver, the airway epithelium and solid tumors in animal models and human xenografts in immuno-deficient mice (Bout 1996, 1997; Blaese *et al,* 1995).

Gene transfer vectors derived from adenoviruses (adenoviral vectors) have a number of features that make them particularly useful for gene transfer:
1) the biology of the adenoviruses is well characterized,
2) the adenovirus is not associated with severe human pathology,
3) the virus is extremely efficient in introducing its DNA into the host cell,
4) the virus can infect a wide variety of cells and has a broad host-range,
5) the virus can be produced at high titers in large quantities,
6) and the virus can be rendered replication defective by deletion of the early-region 1 (E1) of the viral genome (Brody and Crystal, 1994).

However, there is still a number of drawbacks associated with the use of adenoviral vectors:
1) Adenoviruses, especially the well investigated serotypes Ad2 and Ad5 usually elicit an immune response by the host into which they are introduced,
2) it is currently not feasible to target the virus to certain cells and tissues,
3) the replication and other functions of the adenovirus are not always very well suited for the cells, which are to be provided with the additional genetic material,
4) the serotypes Ad2 or Ad5, are not ideally suited for delivering additional genetic material to organs other than the liver. The liver can be particularly well transduced with vectors derived from Ad2 or Ad5. Delivery of such vectors via the bloodstream leads to a significant delivery of the vectors to the cells of the liver. In therapies where other cell types then liver cells need to be transduced some means of liver exclusion must be applied to prevent uptake of the vector by these cells. Current methods rely on the physical separation of the vector from the liver cells, most of these methods rely on localizing the vector and/or the target organ via surgery, balloon angioplasty or direct injection into an organ via for instance needles. Liver exclusion is also being practiced through delivery of the vector to compartments in the body that are essentially isolated from the bloodstream thereby preventing transport of the vector to the liver. Although these methods mostly succeed in avoiding gross delivery of the vector to the liver, most of the methods are crude and still have considerable leakage and/or have poor target tissue penetration characteristics. In some cases inadvertent delivery of the vector to liver cells can be toxic to the patient. For instance, delivery of a herpes simplex virus (HSV) thymidine kinase (TK) gene for the subsequent killing of dividing cancer cells through administration of gancyclovir is quite dangerous when also a significant amount of liver cells are transduced by the vector. Significant delivery and subsequent expression of the HSV-TK gene to liver cells is associated with severe toxicity. Thus there is a discrete need for an inherently safe vector provided with the property of a reduced transduction efficiency of liver cells.

### BRIEF DESCRIPTION OF DRAWINGS

Table I: Oligonucleotides and degenerate oligonucleotides used for the amplification of DNA encoding fiber proteins derived from alternative adenovirus serotypes. (Bold letters represent NdeI restriction site (A-E), NsiI restriction site (1-6, 8), or PacI restriction site, (7).
Table II: Biodistribution of chimeric adenovirus upon intravenous tail vein injection. Values represent luciferase activity/ µg of total protein. All values below 200 Relative light units/ µg protein are considered background. ND = not determined
Table III: Expression of CAR and integrins on the cell surface of endothelial cells and smooth muscle cells. 70%: Cells harvested for FACS analysis at a cell density of 70% confluency. 100%: Cells harvested for FACS analysis at a cell density of 100% confluency. PER.C6 cells were taken as a control for antibody staining. Values represent percentages of cells that express CAR or either one of the integrins at levels above background. As background control, HUVECs or HUVsmc were incubated only with the secondary, rat-anti-mouse IgG1-PE labeled antibody.
Table IV: Determination of transgene expression (luciferase activity) per µg of total cellular protein after infection of A549 cells.

- Figure 1:: Schematic drawing of the pBr/Ad.Bam-rITR construct.
- Figure 2:: Schematic drawing of the strategy used to delete the fiber gene from the pBr/Ad.Bam-rITR construct.
- Figure 3:: Schematic drawing of construct pBr/Ad.BamRΔfib.
- Figure 4:: Sequences of the chimaeric fibers Ad5/12, Ad5/16, Ad5/28, and Ad5/40-L.
- Figure 5:: Schematic drawing of the construct pClipsal-Luc.
- Figure 6:: Schematic drawing of the method to generate chimaeric adenoviruses using three overlapping fragments. Early (E) and late regions (L) are indicated. L5 is the fiber coding sequence.

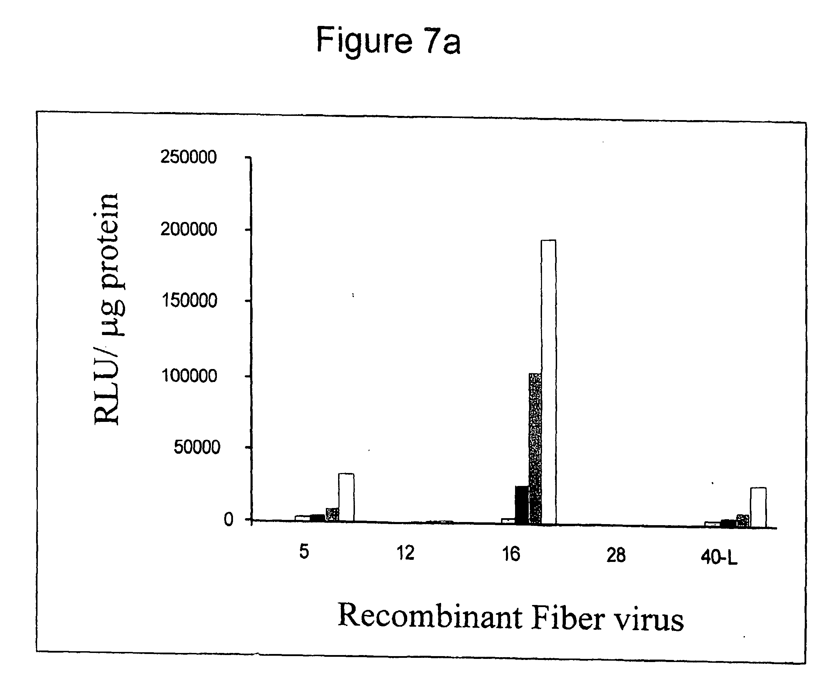

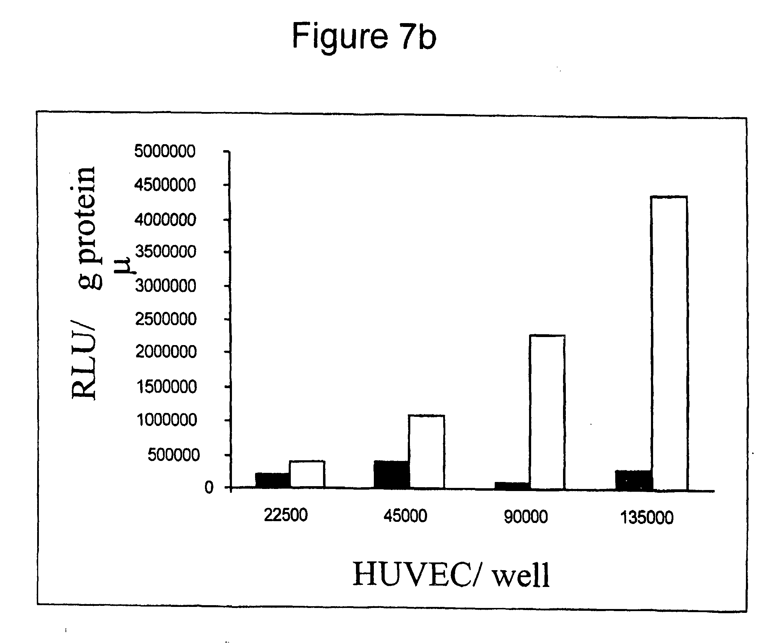

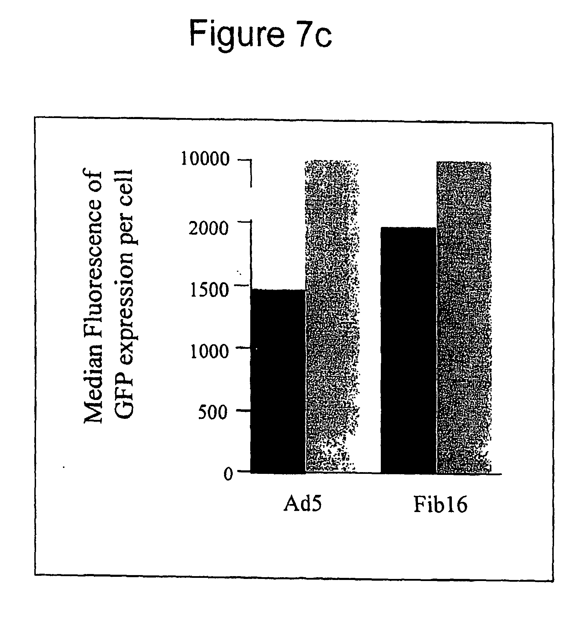

- Figure 7:: A) Infection of HUVEC cells using different amounts of virus particles per cell and different fiber chimeric adenoviruses. Virus concentration: 10000 vp/ cell (= white bar), 5000 vp/ cell (= grey bar), 2500 vp/ cell (= Black bar) 1000 vp/ cell (light grey bar, 250 and 50 vp/ cell no detectable luciferase activity above background. Luciferase activity is expressed in relative light units (RLU) per microgram cellular protein. B) Infection of HUVEC cells using different concentrations of cells (22500, 45000, 90000, or 135000 cells seeded per well) and either adenovirus serotype 5 (black bar) or the fiber 16 chimeric adenovirus (white bar). Luciferase activity is expressed in relative light units (RLU) per microgram cellular protein. C) Flow cytometric analysis on Human aorta EC transduced with 500 (Black bar) or 5000 (grey bar) virus particles per cell of Ad5 or the fiber 16 chimeric virus (Fib16). Non-infected cells were used to set the background at 1% and a median fluorescence of 5.4. The maximum shift in the median fluorescence that can be observed on a flow cytometer is 9999. This latter indicates that at 5000 vp/ cell both Ad5 and Fibl6 are outside the sensitivity scale of the flow cytometer.

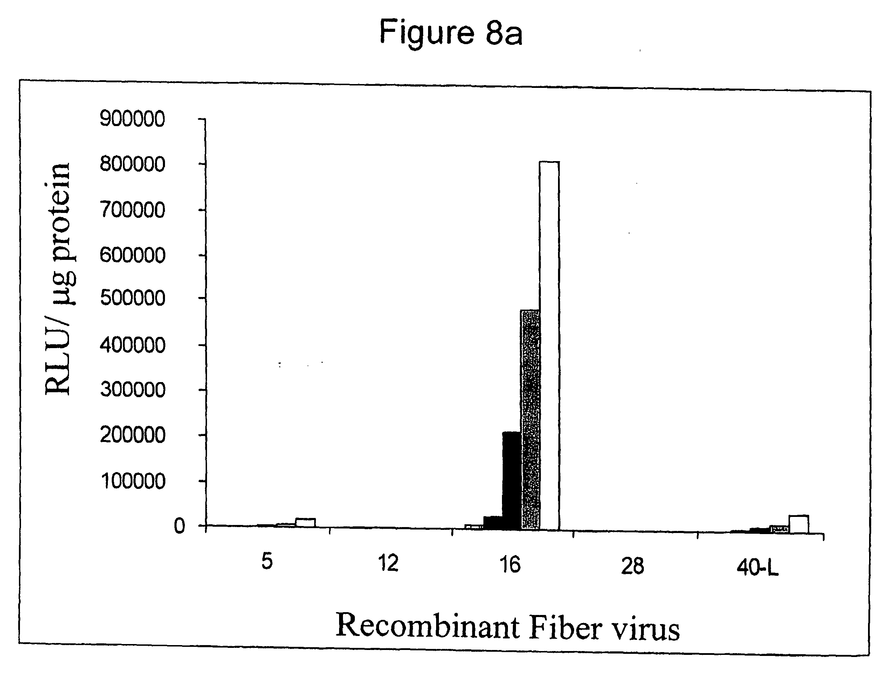

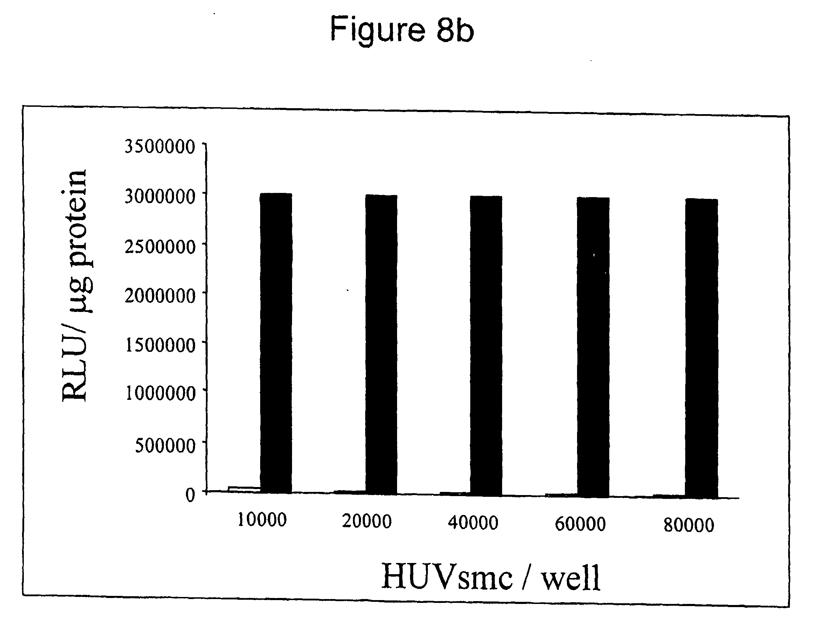

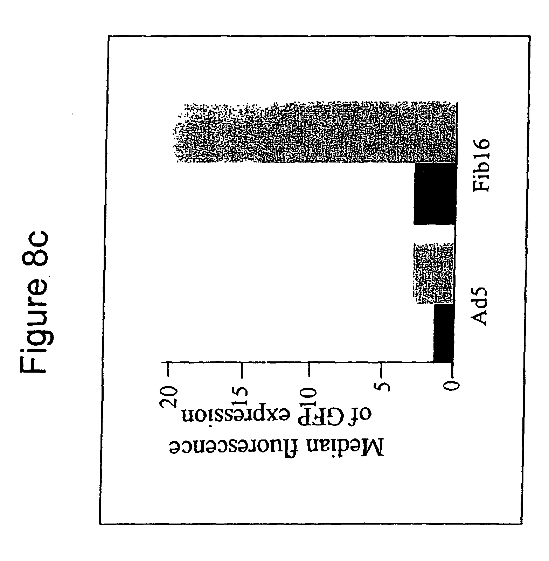

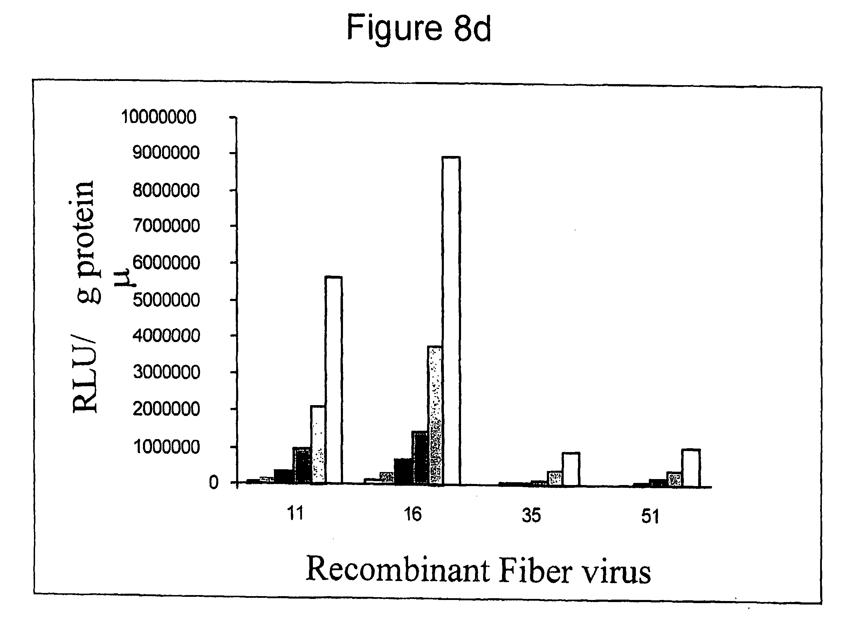

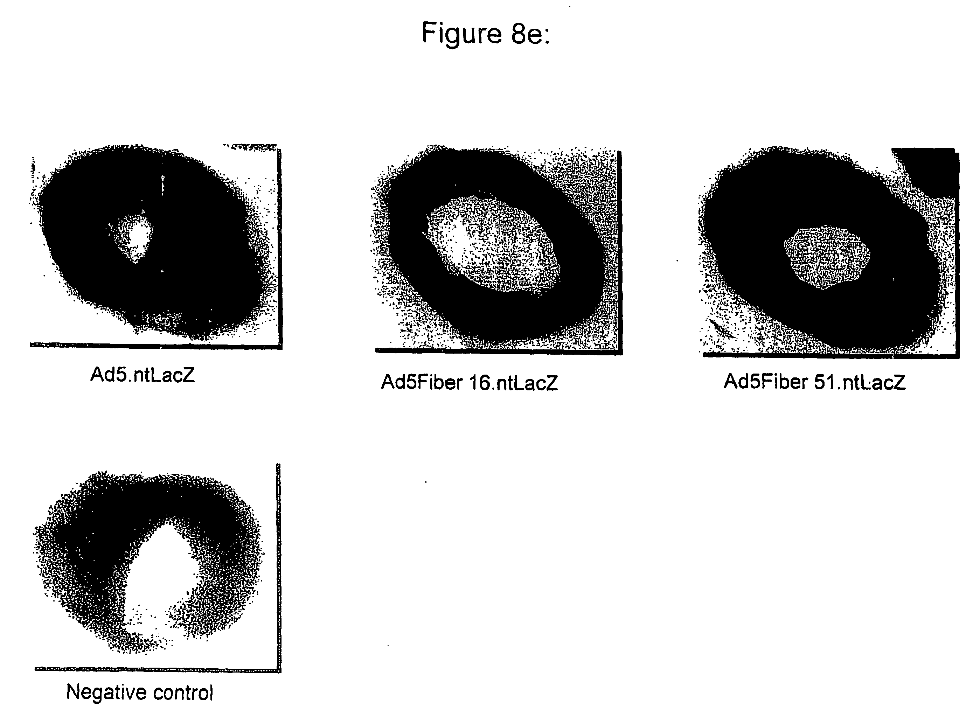

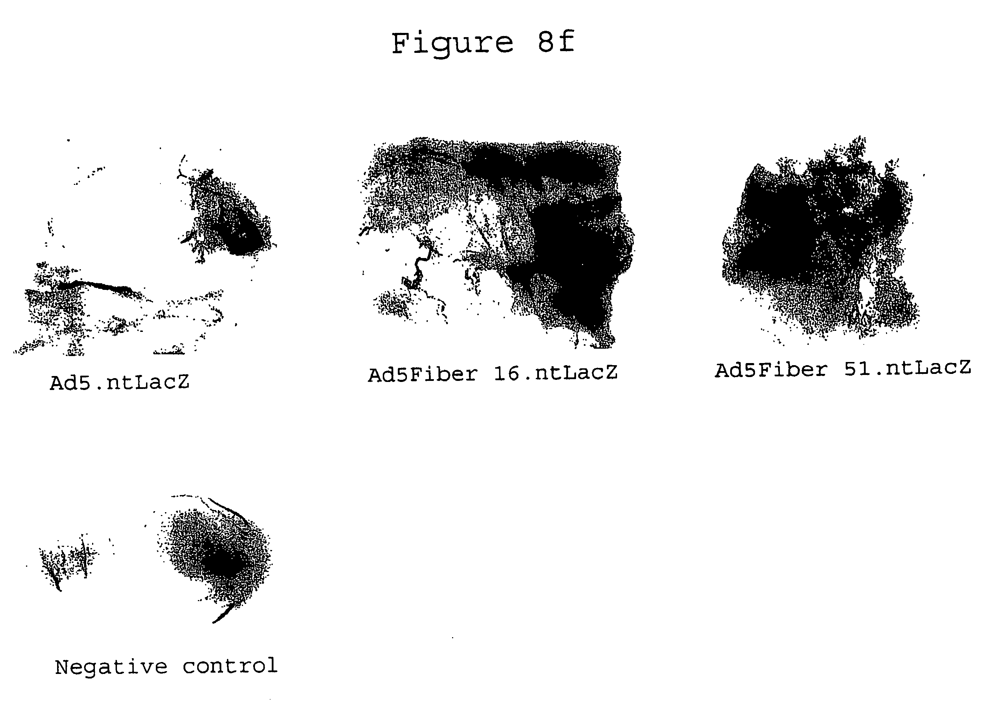

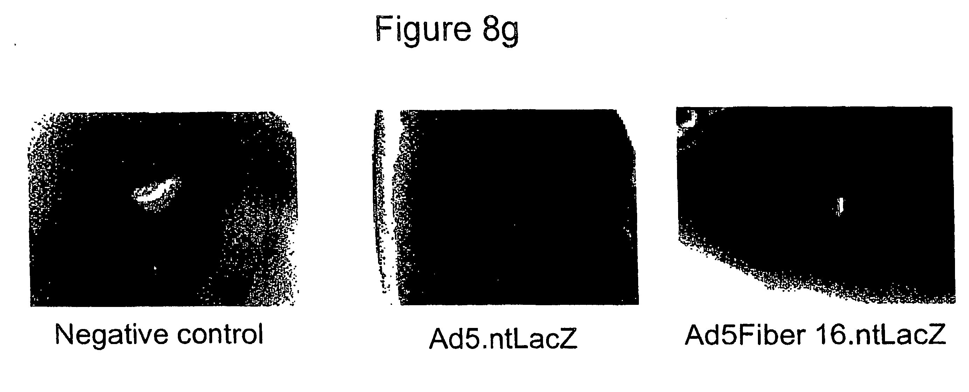

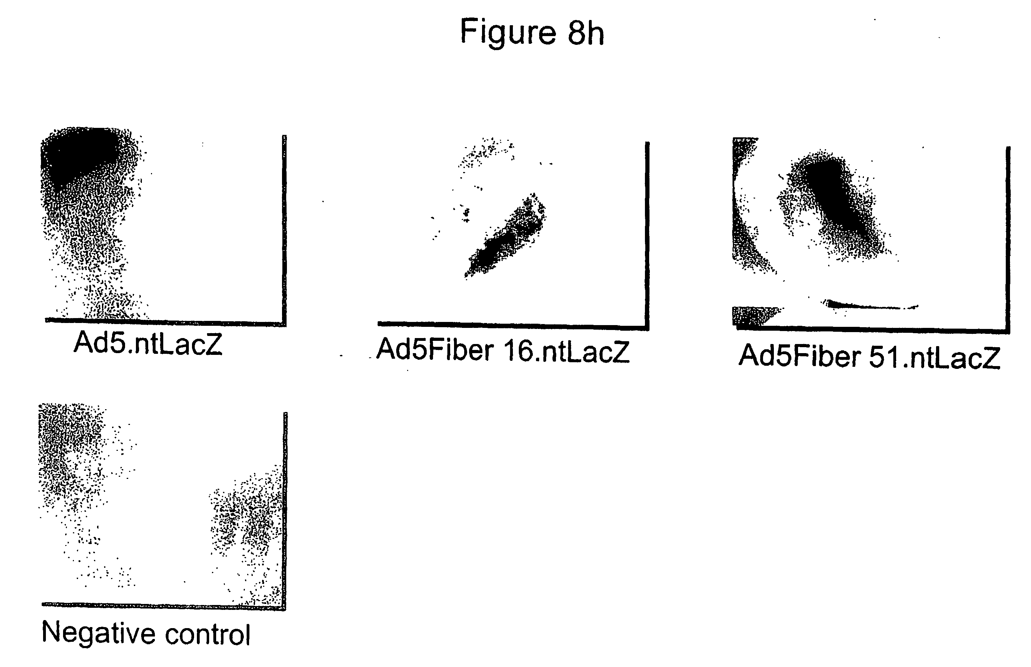

- Figure 8:: A) Infection of HUVsmc cells using different amounts of virus particles per cell and different fiber mutant Ad5 based adenoviruses. Virus concentration: 5000 vp/ cell (= white bar), 2500 vp/ cell (= grey bar), 1250 vp/ cell (= dark grey bar), 250 vp/ cell (= black bar), or 50 vp/ cell (light grey bar). Luciferase activity is expressed as relative light units (RLU) per microgram cellular protein. B) Infection of HUVsmc cells using different concentrations of cells (10000, 20000, 40000, 60000, or 80000 cells per well) and either adenovirus serotype 5 (white bars) or the fiber 16 chimeric adenovirus (black bars). A plateau is observed after infection with chimeric fiber 16 adenovirus due to the fact that transgene expression is higher than the sensitivity range of the bioluminimeter used. C) Human umbilical vein SMC transduced with 500 VP/ cell (black bar) or 5000 VP/ cell (grey bar) using either Ad5 or the fiber 16 mutant (Fib16). Non-transduced cells were used to set a background median fluorescence of approximately 1. Shown is the median fluorescence of GFP expression as measured by flow cytometry. D) HUVsmc were infected with 312 (light grey bar), 625 (grey bar), 1250 (black bar), 2500 (dark grey bar), 5000 (light grey bar), or 10000 (white bar) virus particles per cell of either the fiber 11, 16, 35, or 51 chimeric virus. Luciferase transgene expression expressed as relative light units (RLU) per microgram protein was measured 48 hours after virus exposure. E) Macroscopic photographs of LacZ staining on saphenous samples. Nuclear targeted LacZ (ntLacZ) yields a deep blue color which appears black or dark grey in non-color prints. F) Macroscopic photographs of LacZ staining on pericard samples. Nuclear targeted LacZ (ntLacZ) gives a deep blue color which appears black in non-color prints G) Macroscopic photographs of LacZ staining on right coronary artery samples. Nuclear targeted LacZ (ntLacZ) gives a deep blue color which appears black in non-color prints H) LacZ staining on Left artery descending (LAD) samples. Nuclear targeted LacZ (ntLacZ) gives a deep blue color which appears black in non-color prints
- Figure 9:: Sequences including the gene encoding adenovirus 16 fiber protein as published in Genbank and sequences including a gene encoding a fiber from an adenovirus 16 variant as isolated in the present invention, wherein the sequences of the fiber protein are from the NdeI-site. Figure 9A nucleotide sequence comparison. Figure 9B amino-acid comparison.

### SUMMARY OF THE INVENTION

The present invention provides gene therapy methods, compounds and medicines. The present invention is particularly useful in gene therapy applications where endothelial cells and /or smooth muscle cells form the target cell type. The present invention relates to gene delivery vehicles provided with a tissue tropism for at least endothelial cells and /or smooth muscle cells. The present invention further relates to gene delivery vehicles having been deprived of a tissue tropism for liver cells.

### DETAILED DESCRIPTION OF THE INVENTION.

It is an object of the current invention to provide materials and methods to overcome the limitations of adenoviral vectors mentioned above. In a broad sense, the invention provides new adenoviruses, derived in whole or in part from serotypes different from Ad5. Specific genes of serotypes with preferred characteristics may be combined in a chimaeric vector to give rise to a vector that is better suited for specific applications. Preferred characteristics include, but are not limited to, improved infection of a specific target cell, reduced infection of non-target cells, improved stability of the virus, reduced uptake in antigen presenting cells (APC), or increased uptake in APC, reduced toxicity to target cells, reduced neutralization in humans or animals, reduced or increased CTL response in humans or animals, better and/or prolonged transgene expression, increased penetration capacity in tissues, improved yields in packaging cell lines, etc.

One aspect of the present invention facilitates the combination of the low immunogenicity of some adenoviruses with the characteristics of other adenoviruses that allow efficient gene therapy. Such characteristics may be a high specificity for certain host cells, a good replication machinery for certain cells, a high rate of infection in certain host cells, low infection efficiency in non-target cells, high or low efficiency of APC infection, etc. The invention thus may provide chimaeric adenoviruses having the useful properties of at least two adenoviruses of different serotypes.
Typically, two or more requirements from the above non-exhaustive list are required to obtain an adenovirus capable of efficiently transferring genetic material to a host cell. Therefore the present invention provides adenovirus derived vectors which can be used as cassettes to insert different adenoviral genes from different adenoviral serotypes at the required sites. This way one can obtain a vector capable of producing a chimaeric adenovirus, whereby of course also a gene of interest can be inserted (for instance at the site of E1 of the original adenovirus). In this manner the chimaeric adenovirus to be produced can be adapted to the requirements and needs of certain hosts in need of gene therapy for certain disorders. To enable this virus production, a packaging cell will generally be needed in order to produce sufficient amount of safe chimaeric adenoviruses.
In one of its aspects the present invention provides adenoviral vectors comprising at least a fragment of a fiber protein of an adenovirus from subgroup B. Said fiber protein may be the native fiber protein of the adenoviral vector or may be derived from a serotype different from the serotype the adenoviral vector is based on. In the latter case the adenoviral vector according to the invention is a chimaeric adenovirus displaying at least a fragment of the fiber protein derived from subgroup B adenoviruses that fragment comprising at least the receptor binding sequence. Typically such a virus will be produced using a vector (typically a plasmid, a cosmid or baculovirus vector). Such vectors are also subject of the present invention. A preferred vector is a vector that can be used to make a chimaeric recombinant virus specifically adapted to the host to be treated and the disorder to be treated.
The present invention also provides a chimaeric adenovirus based on adenovirus type 5 but having at least a fragment of the fiber sequence from adenovirus type 16, whereby the fragment of the fiber of Ad16 comprises the fragment of the fiber protein that is involved in binding a host cell. The present invention also provides chimaeric adenoviral vectors that show improved infection as compared to adenoviruses from other subgroups in specific host cells for example, but not limited to, endothelial cells and smooth muscle cells of human or animal origin. An important feature of the present invention is the means to produce the chimaeric virus. Typically, one does not want an adenovirus batch to be administered to the host cell, which contains replication competent adenovirus. In general therefore it is desired to omit a number of genes (but at least one) from the adenoviral genome on the vector encoding the chimaeric virus and to supply these genes in the genome of the cell in which the vector is brought to produce chimaeric adenovirus. Such a cell is usually called a packaging cell. The invention thus also provides a packaging cell for producing a chimaeric adenovirus according to the invention, comprising in trans all elements necessary for adenovirus production not present on the adenoviral vector according to the invention. Typically vector and packaging cell have to be adapted to one another in that they have all the necessary elements, but that they do not have overlapping elements which lead to replication competent virus by recombination. Thus the invention also provides a kit of parts comprising a packaging cell according to the invention and a recombinant vector according the invention whereby there is essentially no sequence overlap leading to recombination resulting in the production of replication competent adenovirus between said cell and said vector. For certain applications for example when the therapy is aimed at eradication of tumor cells, the adenoviral vector according to the invention may be replication competent or capable of replicating under certain conditions for example in specific cell types like tumor cells or tumor endothelial cells.
It is within the scope of the invention to insert more genes, or a functional part of these genes from the same or other serotypes into the adenoviral vector replacing the corresponding native sequences. Thus for example replacement of (a functional part of the) fiber sequences with corresponding sequences of other serotypes may be combined with for example replacements of (a functional part of) other capsid genes like penton base or hexon with corresponding sequences of said serotype or of other distinct serotypes. Persons skilled in the art understand that other combinations not limited to the said genes are possible and are within the scope of the invention. The chimaeric adenoviral vector according to the invention may originate from at least two different serotypes. This may provide the vector with preferred characteristics such as improved infection of target cells and/or less infection of non-target cells, improved stability of the virus, reduced immunogenicity in humans or animals (e.g. reduced uptake in APC, reduced neutralization in the host and/or reduced cytotoxic T-lymphocyte (CTL) response), increased penetration of tissue, better longevity of transgene expression, etc. In this aspect it is preferred to use capsid genes e.g. penton and/or hexon genes from less immunogenic serotypes as defined by the absence or the presence of low amounts of neutralizing antibodies in the vast majority of hosts. It is also preferred to use fiber and/or penton sequences from serotypes that show improved binding and internalization in the target cells. Furthermore it is preferred to delete from the viral vector those genes which lead to expression of adenoviral genes in the target cells. In this aspect a vector deleted of all adenoviral genes is also preferred. Furthermore it is preferred that the promoter driving the gene of interest to be expressed in the target cells is a cell type specific promoter.

In order to be able to precisely adapt the viral vector and provide the chimaeric virus with the desired properties at will, it is preferred that a library of adenoviral genes is provided whereby the genes to be exchanged are located on plasmid- or cosmid-based adenoviral constructs whereby the genes or the sequences to be exchanged are flanked by restriction sites. The preferred genes or sequences can be selected from the library and inserted in the adenoviral constructs that are used to generate the viruses. Typically such a method comprises a number of restriction and ligation steps and transfection of a packaging cell. The adenoviral vector can be transfected in one piece, or as two or more overlapping fragments, whereby viruses are generated by homologous recombination. For example the adenoviral vector may be built up from two or more overlapping sequences for insertion or replacements of a gene of interest in for example the E1 region, for insertion or replacements in penton and/or hexon sequences, and for insertions or replacements into fiber sequences. Thus the invention provides a method for producing chimaeric adenoviruses having one or more desired properties like a desired host range and diminished antigenicity, comprising providing one or more vectors according to the invention having the desired insertion sites, inserting into said vectors at least a functional part of a fiber protein derived from an adenovirus serotype having the desired host range and/or inserting a functional part of a capsid protein derived from an adenovirus serotype having relatively low antigenicity and transfecting said vectors in a packaging cell according to the invention and allowing for production of chimaeric viral particles. Of course other combinations of other viral genes originating from different serotypes can also be inserted as disclosed herein before. Chimaeric viruses having only one non-native sequence in addition to an insertion or replacement of a gene of interest in the E1 region, are also within the scope of the invention. An immunogenic response to adenovirus that typically occurs is the production of neutralizing antibodies by the host. This is typically a reason for selecting a capsid protein like penton, hexon and/or fiber of a less immunogenic serotype.
Of course it may not be necessary to make chimaeric adenoviruses which have complete proteins from different serotypes. It is well within the skill of the art to produce chimaeric proteins, for instance in the case of fiber proteins it is very well possible to have the base of one serotype and the shaft and the knob from another serotype. In this manner it becomes possible to have the parts of the protein responsible for assembly of viral particles originate from one serotype, thereby enhancing the production of intact viral particles. Thus the invention also provides a chimaeric adenovirus according to the invention, wherein the hexon, penton, fiber and/or other capsid proteins are chimaeric proteins originating from different adenovirus serotypes. Besides generating chimaeric adenoviruses by swapping entire wild type capsid (protein) genes etc. or parts thereof, it is also within the scope of the present invention to insert capsid (protein) genes etc. carrying non-adenoviral sequences or mutations such as point mutations, deletions, insertions, etc. which can be easily screened for preferred characteristics such as temperature stability, assembly, anchoring, redirected infection, altered immune response etc. Again other chimaeric combinations can also be produced and are within the scope of the present invention.

It has been demonstrated in mice and rats that upon in vivo systemic delivery of recombinant adenovirus of common used serotypes for gene therapy purposes more than 90% of the virus is trapped in the liver (Herz et al, 1993; Kass-Eisler et al, 1994; Huard et al, 1995). It is also known that human hepatocytes are efficiently transduced by adenovirus serotype 5 vectors (Castell, J.V., Hernandez, D. Gomez-Foix, A.M., Guillen, I, Donato, T. and Gomez-Lechon, M.J. (1997). Adenovirus-mediated gene transfer into human hepatocytes: analysis of the biochemical functionality of transduced cells. Gene Ther. 4(5), p455-464). Thus in vivo gene therapy by systemic delivery of Ad2 or Ad5 based vectors is seriously hampered by the efficient uptake of the viruses in the liver leading to unwanted toxicity and less virus being available for transduction of the target cells. Therefore, alteration of the adenovirus serotype 5 host cell range to be able to target other organs in vivo is a major interest of the invention.

To obtain re-directed infection of recombinant adenovirus serotype 5, several approaches have been or still are under investigation. Wickham et al have altered the RGD (Arg, Gly, Asp) motif in the penton base which is believed to be responsible for the αᵥβ₃ and αᵥβ₅ integrin binding to the penton base. They have replaced this RGD motif by another peptide motif which is specific for the α₄β₁ receptor. In this way targeting the adenovirus to a specific target cell could be accomplished (Wickham et al, 1995). Krasnykh et al (1998) have made use of the HI loop available in the knob. This loop is, based on X-ray crystallography, located on the outside of the knob trimeric structure and therefore is thought not to contribute to the intramolecular interactions in the knob. Insertion of a FLAG coding sequence into the HI loop resulted in fiber proteins that were able to trimerise and it was further shown that viruses containing the FLAG sequence in the knob region could be made. Although interactions of the FLAG-containing knob with CAR are not changed, insertion of ligands in the HI loop may lead to retargeting of infection. Although successful introduction of changes in the adenovirus serotype 5 fiber and penton-base have been reported, the complex structure of knob and the limited knowledge of the precise amino acids interacting with CAR render such targeting approaches laborious and difficult. The use of antibodies binding to CAR and to a specific cellular receptor has also been described (Wickham et al, 1996; Rogers et al, 1997). This approach is however limited by the availability of a specific antibody and by the complexity of the gene therapy product.
To overcome the limitations described above we used pre-existing adenovirus fibers, penton base proteins, hexon proteins or other capsid proteins derived from other adenovirus serotypes. By generating chimaeric adenovirus serotype 5 libraries containing structural proteins of alternative adenovirus serotypes, we have developed a technology, which enables rapid screening for a recombinant adenoviral vector with preferred characteristics.

It is an object of the present invention to provide methods for the generation of chimaeric capsids which can be targeted to specific cell types *in vitro* as well as *in vivo,* and thus have an altered tropism for certain cell types. It is a further object of the present invention to provide methods and means by which an adenovirus or an adenovirus capsid can be used as a protein or nucleic acid delivery vehicle to a specific cell type or tissue.
The generation of chimaeric adenoviruses based on adenovirus serotype 5 with modified late genes is described. For this purpose, three plasmids, which together contain the complete adenovirus serotype 5 genome, were constructed. From one of these plasmids part of the DNA encoding the adenovirus serotype 5 fiber protein was removed and replaced by linker DNA sequences that facilitate easy cloning. This plasmid subsequently served as template for the insertion of DNA encoding fiber protein derived from different adenovirus serotypes. The DNA sequences derived from the different serotypes were obtained using the polymerase chain reaction technique in combination with (degenerate) oligonucleotides. At the former E1 location in the genome of adenovirus serotype 5, any gene of interest can be cloned. A single transfection procedure of the three plasmids together results in the formation of a recombinant chimaeric adenovirus. Alternatively, cloning of the sequences obtained from the library of genes can be such that the chimaeric adenoviral vector is build up from one or two fragments. For example one construct contains at least the left ITR and sequences necessary for packaging of the virus, an expression cassette for the gene of interest and sequences overlapping with the second construct comprising all sequences necessary for replication and virus formation not present in the packaging cell as well as the non-native sequences providing the preferred characteristics. This new technology of libraries consisting of chimaeric adenoviruses thus allows for a rapid screening for improved recombinant adenoviral vectors for *in vitro* and *in vivo* gene therapy purposes.

The use of adenovirus type 5 for in vivo gene therapy is limited by the apparent inability to infect certain cell types e.g. human endothelial cells and smooth muscle cells and the preference of infection of certain organs e.g. liver and spleen. Specifically this has consequences for treatment of cardiovascular diseases like restenosis and pulmonary hypertension. Adenovirus-mediated delivery of human ceNOS (constitutive endothelial nitric oxide synthase) has been proposed as treatment for restenosis after percutaneous transluminal coronary angioplasty (PTCA). Restenosis is characterized by progressive arterial remodeling, extracellular matrix formation and intimal hyperplasia at the site of angioplasty (Schwartz *et al,* 1993; Carter *et al,* 1994; Shi *et al*, 1996). NO is one of the vasoactive factors shown to be lost after PTCA-induced injury to the endothelial barrier (Lloyd Jones and Bloch, 1996). Thus restoration of NO levels after balloon-induced injury by means of adenoviral delivery of ceNOS may prevent restenosis (Varenne *et al*, 1998). Other applications for gene therapy whereby the viruses or chimaeric viruses according to the invention are superior to Ad2 or Ad5 based viruses, given as non-limiting examples, are production of proteins by endothelial cells that are secreted into the blood, treatment of hypertension, preventive treatment of stenosis during vein grafting, angiogenesis, heart failure, renal hypertension and others.

In one embodiment this invention describes adenoviral vectors that are, amongst others, especially suited for gene delivery to endothelial cells and smooth muscle cells important for treatment of cardiovascular disorders. The adenoviral vectors preferably are derived from subgroup B adenoviruses or contain at least a functional part of the fiber protein from an adenovirus from subgroup B comprising at least the cell-binding moiety of the fiber protein. In a further preferred embodiment the adenoviral vectors are chimaeric vectors based on adenovirus type 5 and contain at least a functional part of the fiber protein from adenovirus type 16.
In another embodiment this invention provides adenoviral vectors or chimaeric adenoviral vectors that escape the liver following systemic administration. Preferably these adenoviral vectors are derived from subgroup A, B, D, or F in particular serotypes 12, 16, 28 and 40 or contain at least the cell-binding moiety of the fiber protein derived from said adenoviruses.
It is to be understood that in all embodiments the adenoviral vectors may be derived from the serotype having the desired properties or that the adenoviral vector is based on an adenovirus from one serotype and contains the sequences comprising the desired functions of another serotype, these sequences replacing the native sequences in the said serotype.

In another aspect this invention describes chimaeric adenoviruses and methods to generate these viruses that have an altered tropism different from that of adenovirus serotype 5. For example, viruses based on adenovirus serotype 5 but displaying any adenovirus fiber existing in nature. This chimaeric adenovirus serotype 5 is able to infect certain cell types more efficiently, or less efficiently *in vitro* and *in vivo* than the adenovirus serotype 5. Such cells include but are not limited to endothelial cells, smooth muscle cells, dendritic cells, neuronal cells, glial cells, synovial cells, lung epithelial cells, hemopoietic stem cells, monocytic/macrophage cells, tumor cells, skeletal muscle cells, mesothelial cells, synoviocytes, etc.

In another aspect the invention describes the construction and use of libraries consisting of distinct parts of adenovirus serotype 5 in which one or more genes or sequences have been replaced with DNA derived from alternative human or animal serotypes. This set of constructs, in total encompassing the complete adenovirus genome, allows for the construction of unique chimaeric adenoviruses customized for a certain disease, group of patients or even a single individual.
In all aspects of the invention the chimaeric adenoviruses may, or may not, contain deletions in the E1 region and insertions of heterologous genes linked either or not to a promoter. Furthermore, chimaeric adenoviruses may, or may not, contain deletions in the E3 region and insertions of heterologous genes linked to a promoter. Furthermore, chimaeric adenoviruses may, or may not, contain deletions in the E2 and/or E4 region and insertions of heterologous genes linked to a promoter. In the latter case E2 and/or E4 complementing cell lines are required to generate recombinant adenoviruses. In fact any gene in the genome of the viral vector can be taken out and supplied in trans. Thus, in the extreme situation, chimaeric viruses do not contain any adenoviral genes in their genome and are by definition minimal adenoviral vectors. In this case all adenoviral functions are supplied in trans using stable cell lines and/or transient expression of these genes. A method for producing minimal adenoviral vectors is described in WO97/00326 and is taken as reference herein. In another case Ad/AAV chimaeric molecules are packaged into the adenovirus capsids of the invention. A method for producing Ad/AAV chimaeric vectors is described in EP 97204085.1 and is taken as reference herein. In principle any nucleic acid may be provided with the adenovirus capsids of the invention.

In one embodiment the invention provides a gene delivery vehicle having been provided with at least a tissue tropism for smooth muscle cells and/or endothelial cells. In another embodiment the invention provides a gene delivery vehicle deprived of a tissue tropism for at least liver cells. Preferably, said gene delivery vehicle is provided with a tissue tropism for at least smooth muscle cells and/or endothelial cells and deprived of a tissue tropism for at least liver cells. In a preferred embodiment of the invention said gene delivery vehicle is provided with a tissue tropism for at least smooth muscle cells and/or endothelial cells and/or deprived of a tissue tropism for at least liver cells using a fiber protein derived from a subgroup B adenovirus, preferably of adenovirus 16. In a preferred aspect of the invention said gene delivery vehicle comprises a virus capsid. Preferably said virus capsid comprises a virus capsid derived in whole or in part from an adenovirus of subgroup B, preferably from adenovirus 16, or it comprises proteins, or parts thereof, from an adenovirus of subgroup B, preferably of adenovirus 16. In preferred embodiment of the invention said virus capsid comprises proteins, or fragments thereof, from at least two different viruses, preferably adenoviruses. In a preferred embodiment of this aspect of the invention at least one of said virus is an adenovirus of subgroup B, preferably adenovirus 16.
In a preferred embodiment of the invention said gene delivery vehicle comprises an adenovirus fiber protein or fragments thereof. Said fiber protein is preferably derived from an adenovirus of subgroup B, preferably of adenovirus 16. Said gene delivery vehicle may further comprise other fiber proteins, or fragments thereof, from other adenoviruses. Said gene delivery vehicle may or may not comprise other adenovirus proteins. Nucleic acid may be linked directly to fiber proteins, or fragments thereof, but may also be linked indirectly. Examples of indirect linkages include but are not limited to, packaging of nucleic acid into adenovirus capsids or packaging of nucleic acid into liposomes, wherein a fiber protein, or a fragment thereof, is incorporated into an adenovirus capsid or linked to a liposome. Direct linkage of nucleic acid to a fiber protein, or a fragment thereof, may be performed when said fiber protein, or a fragment thereof, is not part of a complex or when said fiber protein, or a fragment thereof, is part of complex such as an adenovirus capsid.
In one embodiment of the invention is provided a gene delivery vehicle comprising an adenovirus fiber protein wherein said fiber protein comprises a tissue determining fragment of an adenovirus of subgroup B adenovirus preferably of adenovirus 16. Adenovirus fiber protein comprises three functional domains. One domain, the base, is responsible for anchoring the fiber to a penton base of the adenovirus capsid. Another domain, the knob, is responsible for receptor recognition whereas the shaft domain functions as a spacer separating the base from the knob. The different domains may also have other function. For instance, the shaft is presumably also involved in target cell specificity. Each of the domains mentioned above may be used to define a fragment of a fiber. However, fragments may also be identified in another way. For instance the knob domain comprises of a receptor binding fragment and a shaft binding fragment. The base domain comprises of a penton base binding fragment and a shaft binding fragment. Moreover, the shaft comprises repeated stretches of amino acids. Each of these repeated stretches may be a fragment.
A tissue tropism determining fragment of a fiber protein may be a single fragment of a fiber protein or a combination of fragments of at least one fiber protein, wherein said tissue tropism determining fragment, either alone or in combination with a virus capsid, determines the efficiency with which a gene delivery vehicle can transduce a given cell or cell type, preferably but not necessarily in a positive way. With a tissue tropism for liver cells is meant a tissue tropism for cells residing in the liver, preferably liver parenchyma cells.
A tissue tropism for a certain tissue may be provided by increasing the efficiency with which cells of said tissue are transduced, alternatively, a tissue tropism for a certain tissue may be provided by decreasing the efficiency with which other cells than the cells of said tissue are transduced.

Fiber proteins possess tissue tropism determining properties. The most well described fragment of the fiber protein involved in tissue tropism is the knob domain. However, the shaft domain of the fiber protein also possesses tissue tropism determining properties. However, not all of the tissue tropism determining properties of an adenovirus capsid are incorporated into a fiber protein.
In a preferred embodiment of the invention, a fiber protein derived from a subgroup B adenovirus, preferably adenovirus 16, is combined with the non-fiber capsid proteins from an adenovirus of subgroup C, preferably of adenovirus 5.
In one aspect of the invention is provided a gene delivery vehicle comprising a nucleic acid derived from an adenovirus. In a preferred embodiment of the invention said adenovirus nucleic acid comprises at least one nucleic acid sequence encoding a fiber protein comprising at least a tissue tropism determining fragment of a subgroup B adenovirus fiber protein, preferably of adenovirus 16. In a preferred aspect said adenovirus comprises nucleic acid from at least two different adenoviruses. In a preferred aspect said adenovirus comprises nucleic acid from at least two different adenoviruses wherein at least one nucleic acid sequence encoding a fiber protein comprises at least a tissue tropism determining fragment of a subgroup B adenovirus fiber protein, preferably of adenovirus 16.
In a preferred embodiment of the invention said adenovirus nucleic acid is modified such that the capacity of said adenovirus nucleic acid to replicate in a target cell has been reduced or disabled. This may be achieved through inactivating or deleting genes encoding early region 1 proteins.
In another preferred embodiment said adenovirus nucleic acid is modified such that the capacity of a host immune system to mount an immune response against adenovirus proteins encoded by said adenovirus nucleic acid has been reduced or disabled. This may be achieved through deletion of genes encoding proteins of early region 2 and/or early region 4.
Alternatively, genes encoding early region 3 proteins, may be deleted, or on the contrary, considering the anti-immune system function of some of the proteins encoded by the genes in early region 3, the expression of early region 3 proteins may be enhanced for some purposes. Also, the adenovirus nucleic acid may be altered by a combination of two or more of the specific alterations of the adenovirus nucleic acid mentioned above. It is clear that when essential genes are deleted from the adenovirus nucleic acid, the genes must be complemented in the cell that is going to produce the adenovirus nucleic acid, the adenovirus vector, the vehicle or the chimaeric capsid. The adenovirus nucleic acid may also be modified such that the capacity of a host immune system to mount an immune response against adenovirus proteins encoded by said adenovirus nucleic acid has been reduced or disabled, in other ways then mentioned above, for instance through exchanging capsid proteins, or fragments thereof, by capsid proteins, or fragments thereof, from other serotypes for which humans do not have, or have low levels of, neutralizing antibodies. Another example of this is the exchange of genes encoding capsid proteins with the genes encoding for capsid proteins from other serotypes. Also capsid proteins, or fragments thereof, may be exchanged for other capsid proteins, or fragments thereof, for which individuals are not capable of, or have a low capacity of, raising an immune response against.

An adenovirus nucleic acid may be altered further or instead of one or more of the alterations mentioned above, by inactivating or deleting genes encoding adenovirus late proteins such as but not-limited to, hexon, penton, fiber and/or protein IX.
In a preferred embodiment of the invention all genes encoding adenovirus proteins are deleted from said adenovirus nucleic acid, turning said nucleic acid into a minimal adenovirus vector.
In another preferred embodiment of the invention said adenovirus nucleic acid is an Ad/AAV chimaeric vector, wherein at least the integration means of an adeno-associated virus (AAV) are incorporated into said adenovirus nucleic acid.
In a preferred embodiment of the invention, a vector or a nucleic acid, which may be one and the same or not, according to the invention further comprises at least one non-adenovirus gene. Preferably, at least one of said non-adenovirus gene is selected from the group of genes encoding: an apolipoprotein, a ceNOS, a herpes simplex virus thymidine kinase, an interleukin-3, an interleukin-1α, an (anti)angiogenesis protein such as angiostatin, an anti-proliferation protein, a vascular endothelial growth factor (VGAF), a basic fibroblast growth factor (bFGF),a hypoxia inducible factor 1α (HIF-1α), a PAI-1 or a smooth muscle cell anti-migration protein.
In another aspect, the invention provides a cell for the production of a gene delivery vehicle provided with at least a tissue tropism for smooth muscle cells and/or endothelial cells. In another aspect, the invention provides a cell for the production of a gene delivery vehicle deprived of at least a tissue tropism for liver cells. In another aspect, the invention provides a cell for the production of a gene delivery vehicle provided with at least a tissue tropism for smooth muscle cells and/or endothelial cells and deprived of at least a tissue tropism for liver cells. In a preferred embodiment of the invention said cell is an adenovirus packaging cell, wherein an adenovirus nucleic acid is packaged into an adenovirus capsid. In one aspect of an adenovirus packaging cell of the invention all proteins required for the replication and packaging of an adenovirus nucleic acid, except for the proteins encoded by early region 1, are provided by genes incorporated in said adenovirus nucleic acid. The early region 1 encoded proteins in this aspect of the invention may be encoded by genes incorporated into the cells genomic DNA. In a preferred embodiment of the invention said cell is PER.C6 (ECACC deposit number 96022940). In general, when gene products required for the replication and packaging of adenovirus nucleic acid into adenovirus capsid are not provided by a adenovirus nucleic acid, they are provided by the packaging cell, either by transient transfection, or through stable transformation of said packaging cell. However, a gene product provided by the packaging cell may also be provided by a gene present on said adenovirus nucleic acid. For instance fiber protein may be provided by the packaging cell, for instance through transient transfection, and may be encoded by the adenovirus nucleic acid. This feature can among others be used to generate adenovirus capsids comprising of fiber proteins from two different viruses.
The gene delivery vehicles of the invention are useful for the treatment of cardiovascular disease or disease treatable by nucleic acid delivery to endothelial cells or smooth muscle cells. A non-limiting example of the latter is for instance cancer, where the nucleic acid transferred comprises a gene encoding an anti-angiogenesis protein.
The gene delivery vehicles of the invention may be used as a pharmaceutical for the treatment of said diseases.
Alternatively, gene delivery vehicles of the invention may be used for the preparation of a medicament for the treatment of said diseases.
In one aspect the invention provides an adenovirus capsid with or provided with a tissue tropism for smooth muscle cells and/or endothelial cells wherein said capsid preferably comprises proteins from at least two different adenoviruses and wherein at least a tissue tropism determining fragment of a fiber protein is derived from a subgroup B adenovirus, preferably of adenovirus 16. In another aspect the invention provides an adenovirus capsid deprived of a tissue tropism for liver cells wherein said capsid preferably comprises proteins from at least two different adenoviruses and wherein at least a tissue tropism determining fragment of a fiber protein is derived from a subgroup B adenovirus, preferably of adenovirus 16.
In one embodiment the invention comprises the use of an adenovirus capsid, for the delivery of nucleic acid to smooth muscle cells and/or endothelial cells. In another embodiment the invention comprises the use of an adenovirus capsid, for preventing the delivery of nucleic acid to liver cells. The adenovirus capsids of the invention may be used for the treatment of cardiovascular disease or disease treatable by nucleic acid delivery to endothelial cells or smooth muscle cells. Example of the latter is for instance cancer where the nucleic acid transferred comprises a gene encoding an anti-angiogenesis protein.
The adenovirus capsids of the invention may be used as a pharmaceutical for the treatment of said diseases.
Alternatively, adenovirus capsids of the invention may be used for the preparation of a medicament for the treatment of said diseases.
In another aspect of the invention is provided construct pBr/Ad.BamRΔFib, comprising adenovirus 5 sequences 21562-31094 and 32794-35938.
In another aspect of the invention is provided construct pBr/AdBamRfibl6, comprising adenovirus 5 sequences 21562-31094 and 32794-35938, further comprising an adenovirus 16 gene encoding fiber protein.
In yet another aspect of the invention is provided construct pBr/AdBamR.pac/fibl6, comprising adenovirus 5 sequences 21562-31094 and 32794-35938, further comprising an adenovirus 16 gene encoding fiber protein, and further comprising a unique PacI-site in the proximity of the adenovirus 5 right terminal repeat, in the non-adenovirus sequence backbone of said construct.
In another aspect of the invention is provided construct pWE/Ad.AflIIrITRfibl6 comprising Ad5 sequence 3534-31094 and 32794-35938, further comprising an adenovirus 16 gene encoding fiber protein.
In another aspect of the invention is provided construct pWE/Ad.AflIIrITRDE2Afib16 comprising Ad5 sequences 3534-22443 and 24033-31094 and 32794-35938, further comprising an adenovirus 16 gene encoding fiber protein.
In the numbering of the sequences mentioned above, the number is depicted until and not until plus.

In a preferred embodiment of the invention said constructs are used for the generation of a gene delivery vehicle or an adenovirus capsid with a tissue tropism for smooth muscle cells and/or endothelial cells.
In another aspect the invention provides a library of adenovirus vectors, or gene delivery vehicles which may be one and the same or not, comprising a large selection of non-adenovirus nucleic acids. In another aspect of the invention, adenovirus genes encoding capsid proteins are used to generate a library of adenovirus capsids comprising of proteins derived from at least two different adenoviruses, said adenoviruses preferably being derived from two different serotypes, wherein preferably one serotype is an adenovirus of subgroup B. In a particularly preferred embodiment of the invention a library of adenovirus capsids is generated comprising proteins from at least two different adenoviruses and wherein at least a tissue tropism determining fragment of fiber protein is derived from an adenovirus of subgroup B, preferably of adenovirus 16.

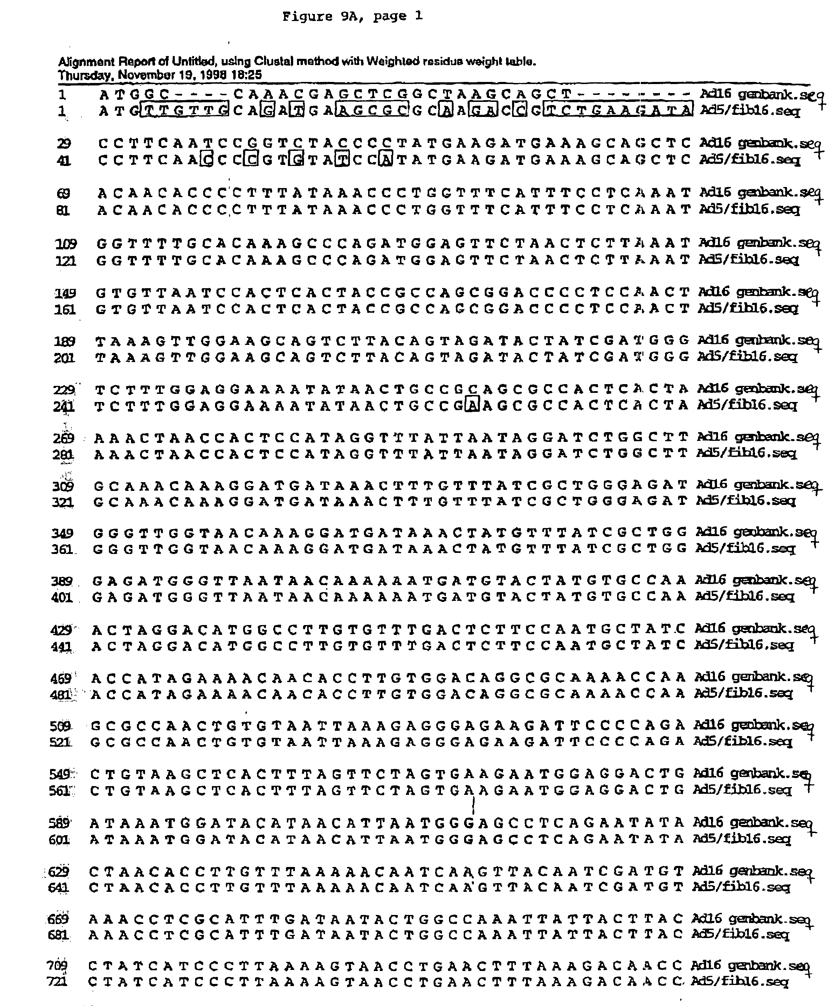

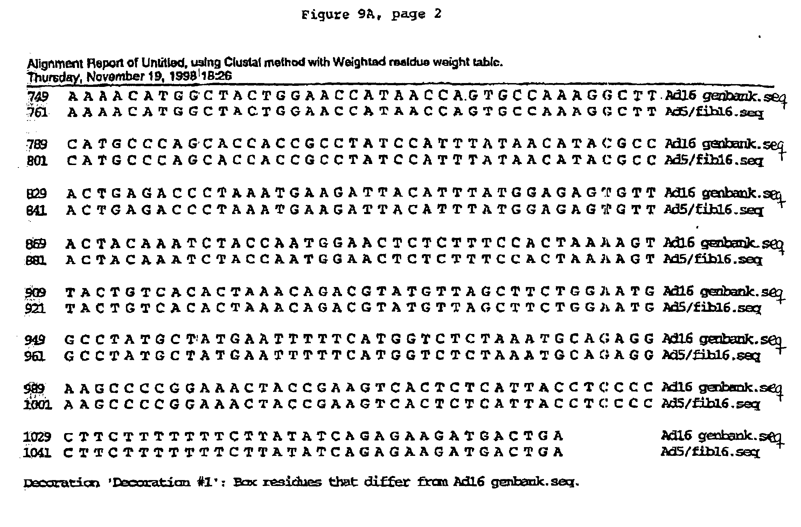

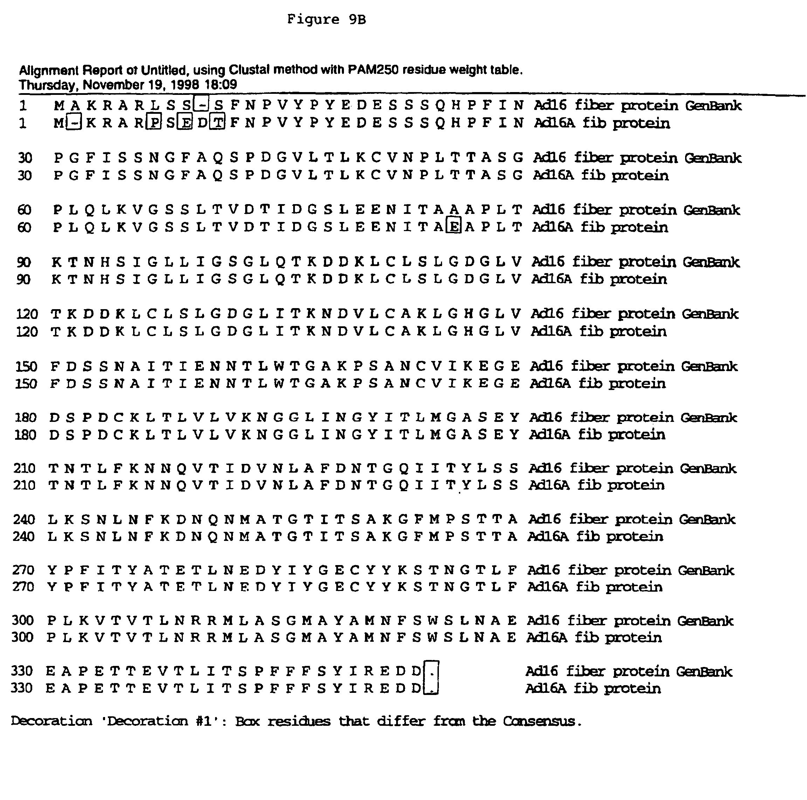

A fiber protein of adenovirus 16 preferably comprises the sequence given in figure 9. However within the scope of the present invention analogous sequences may be obtained through using codon degeneracy. Alternatively, amino-acid substitutions or insertions or deletions may be performed as long as the tissue tropism determining property is not significantly altered. Such amino-acid substitutions may be within the same polarity group or without.

In the following the invention is illustrated by a number of non-limiting examples.

### EXAMPLES

### Example 1: Generation of adenovirus serotype 5 based viruses with chimaeric fiber proteins

### Generation of adenovirus template clones lacking DNA encoding for fiber

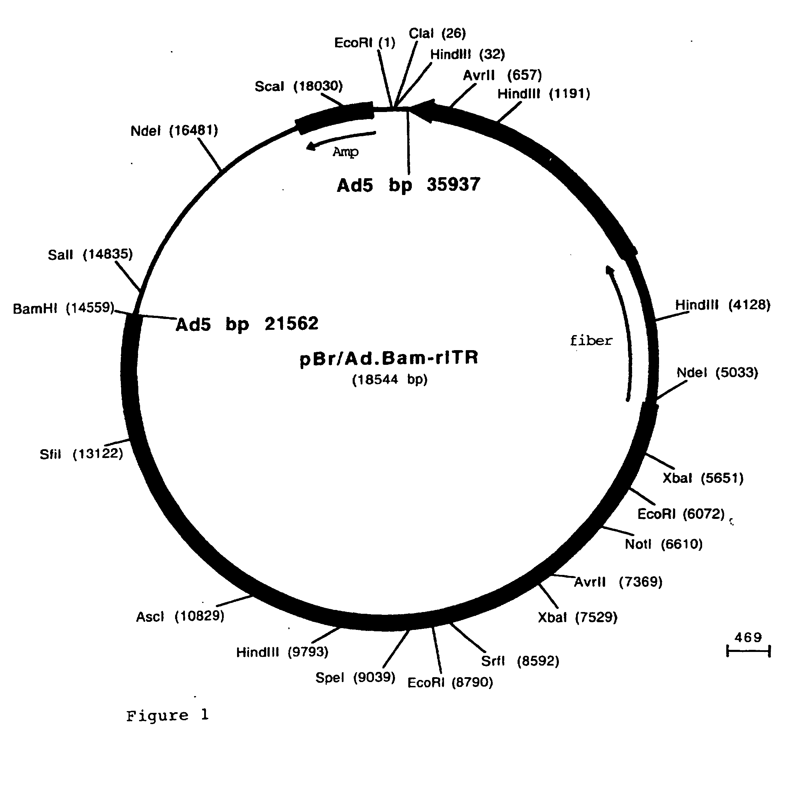

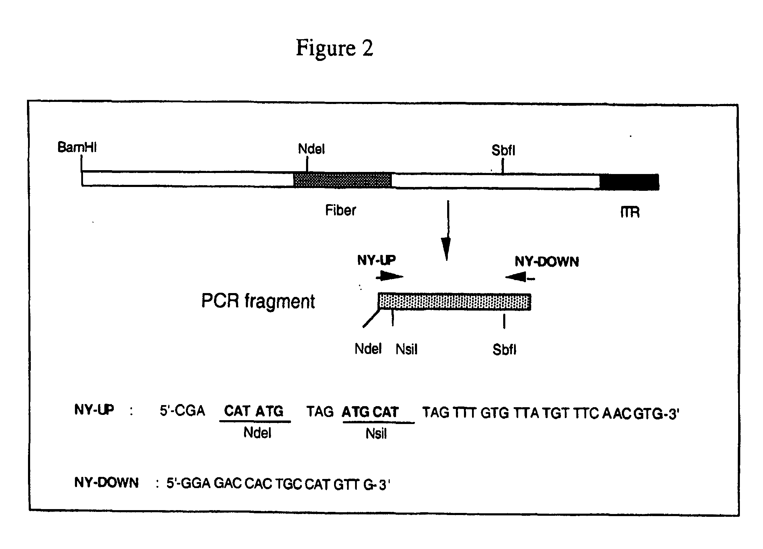

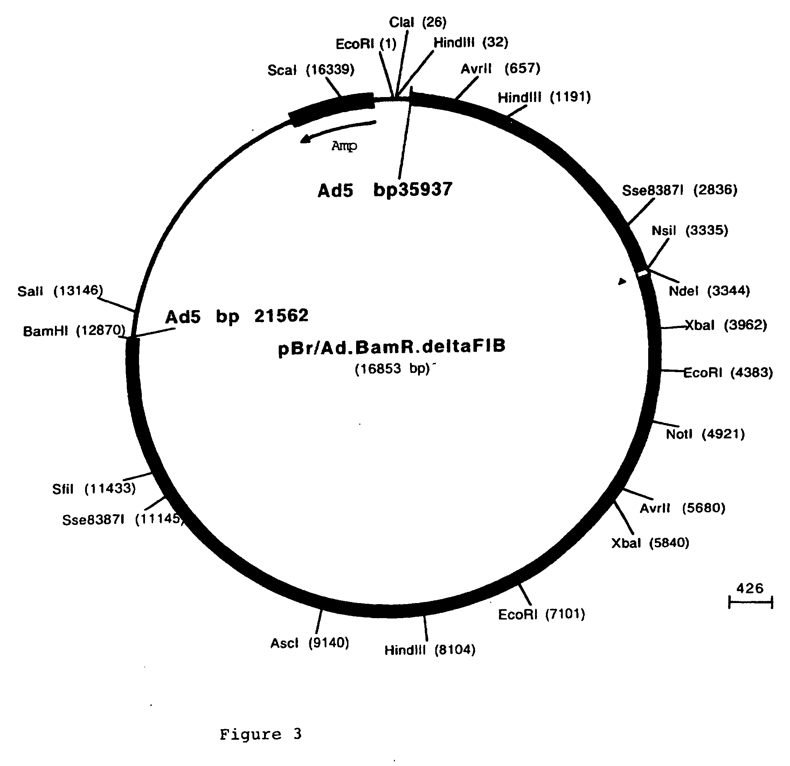

The fiber coding sequence of adenovirus serotype 5 is located between nucleotides 31042 and 32787. To remove the adenovirus serotype 5 DNA encoding fiber we started with construct pBr/Ad.Bam-rITR (Figure 1; ECACC deposit P97082122). From this construct first a NdeI site was removed. For this purpose, pBr322 plasmid DNA was digested with NdeI after which protruding ends were filled using Klenow enzyme. This pBr322 plasmid was then re-ligated, digested with NdeI and transformed into *E. coli* DH5α. The obtained pBr/ΔNdeI plasmid was digested with ScaI and SalI and the resulting 3198 bp vector fragment was ligated to the 15349 bp ScaI-SalI fragment derived from pBr/Ad.BamrITR, resulting in plasmid pBr/Ad.Bam-rITRΔNdeI which hence contained a unique NdeI site. Next a PCR was performed with oligonucleotides "NY-up" and "NY-down" (Figure 2). During amplification, both a NdeI and a NsiI restriction site were introduced to facilitate cloning of the amplified fiber DNAs. Amplification consisted of 25 cycles of each 45 sec. at 94°C, 1 min. at 60°C, and 45 sec. at 72°C. The PCR reaction contained 25 pmol of oligonucleotides NY-up or NY-down, 2mM dNTP, PCR buffer with 1.5 mM MgCl₂, and 1 unit of Elongase heat stable polymerase (Gibco, The Netherlands). One-tenth of the PCR product was run on an agarose gel which demonstrated that the expected DNA fragment of ± 2200 bp was amplified. This PCR fragment was subsequently purified using Geneclean kit system. (Bio101 Inc.) Then, both the construct pBr/Ad.Bam-rITRΔNdeI as well as the PCR product were digested with restriction enzymes NdeI and SbfI. The PCR fragment was subsequently cloned using T4 ligase enzyme into the NdeI and SbfI sites thus generating pBr/Ad.BamRΔFib (Figure 3).

### Amplification of fiber sequences from adenovirus serotypes

To enable amplification of the DNAs encoding fiber protein derived from alternative serotypes degenerate oligonucleotides were synthesized. For this purpose, first known DNA sequences encoding for fiber protein of alternative serotypes were aligned to identify conserved regions in both the tail region as well as the knob region of the fiber protein. From the alignment, which contained the nucleotide sequence of 19 different serotypes representing all 6 subgroups, (degenerate) oligonucleotides were synthesized (see Table I). Also shown in table 3 is the combination of oligonucleotides used to amplify the DNA encoding fiber protein of a specific serotype. The amplification reaction (50 µl) contained 2 mM dNTPs, 25 pmol of each oligonucleotide, standard 1x PCR buffer, 1.5 mM MHCl₂, and 1 Unit Pwo heat stable polymerase (Boehringer Mannheim) per reaction. The cycler program contained 20 cycles, each consisting of 30 sec. 94°C, 60 sec. 60-64°C, and 120 sec. 72°C. One-tenth of the PCR product was run on an agarose gel to demonstrate that a DNA fragment was amplified. Of each different template, two independent PCR reactions were performed.

### Generation of chimaeric adenoviral DNA constructs

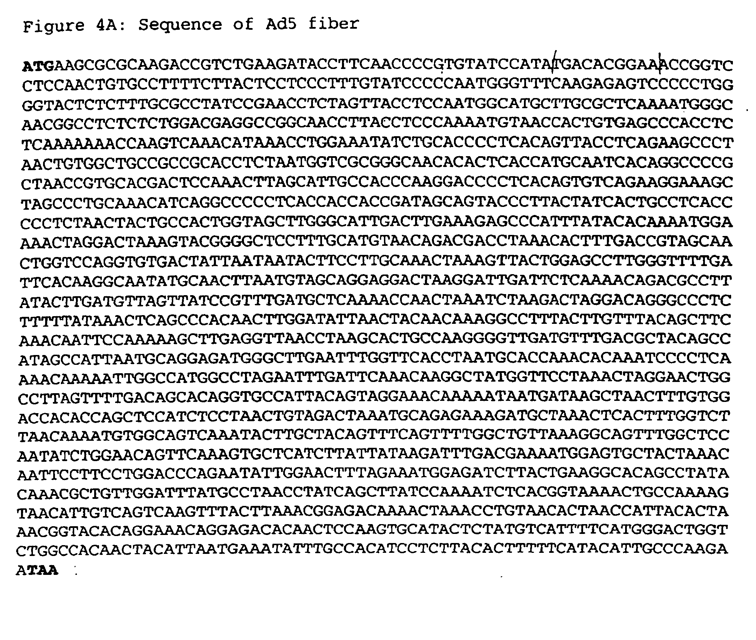

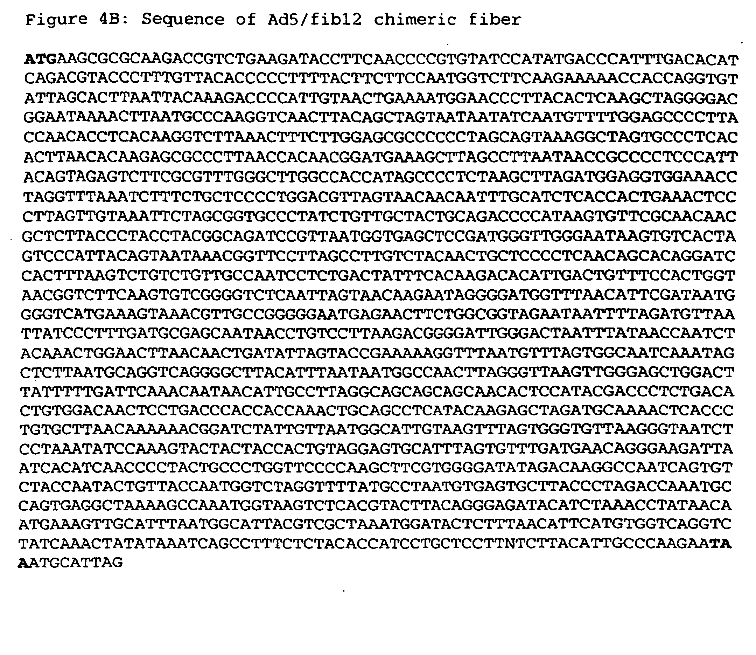

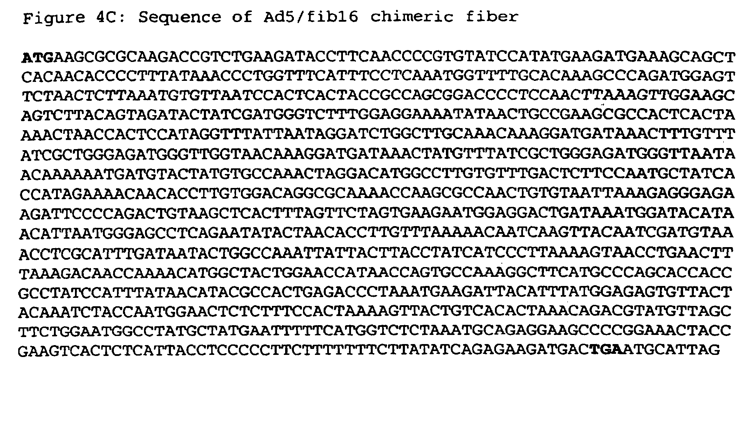

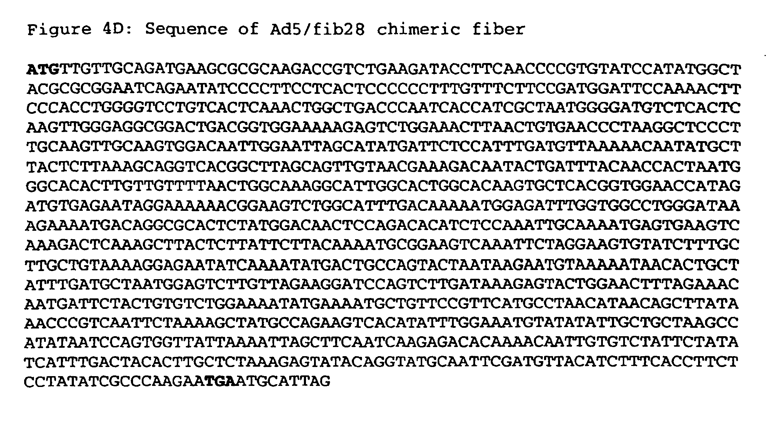

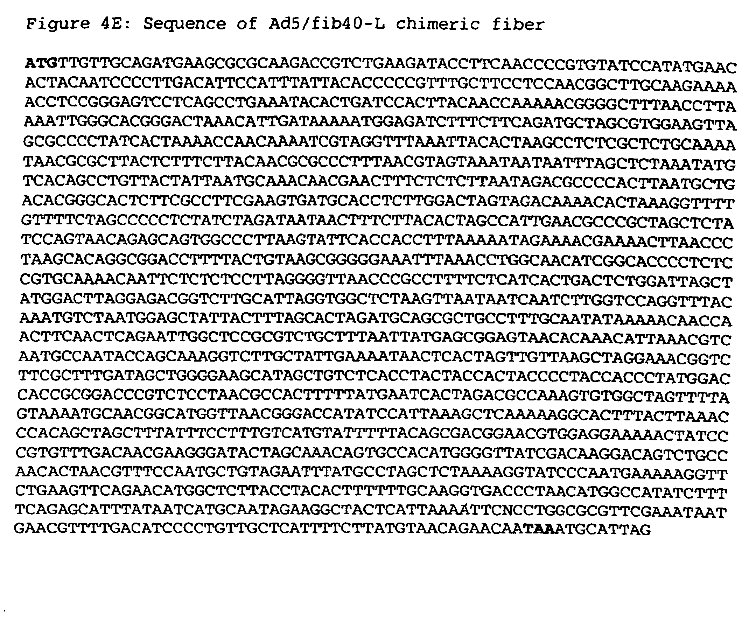

All amplified fiber DNAs as well as the vector (pBr/Ad.BamRΔFib) were digested with NdeI and NsiI. The digested DNAs were subsequently run on an agarose gel after which the fragments were isolated from the gel and purified using the Geneclean kit (Bio101 Inc). The PCR fragments were then cloned into the NdeI and NsiI sites of pBr/AdBamRΔFib, thus generating pBr/AdBamRFibXX (where XX the serotype number of which the fiber DNA was isolated). The inserts generated by PCR were sequenced to confirm correct amplification. The obtained sequences of the different fiber genes are shown in Figure 4.

### Generation of recombinant adenovirus chimaeric for fiber protein

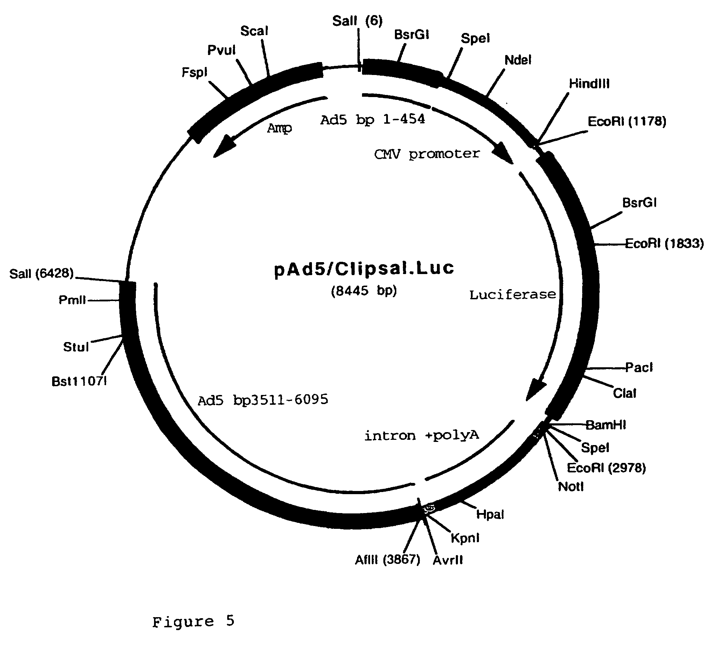

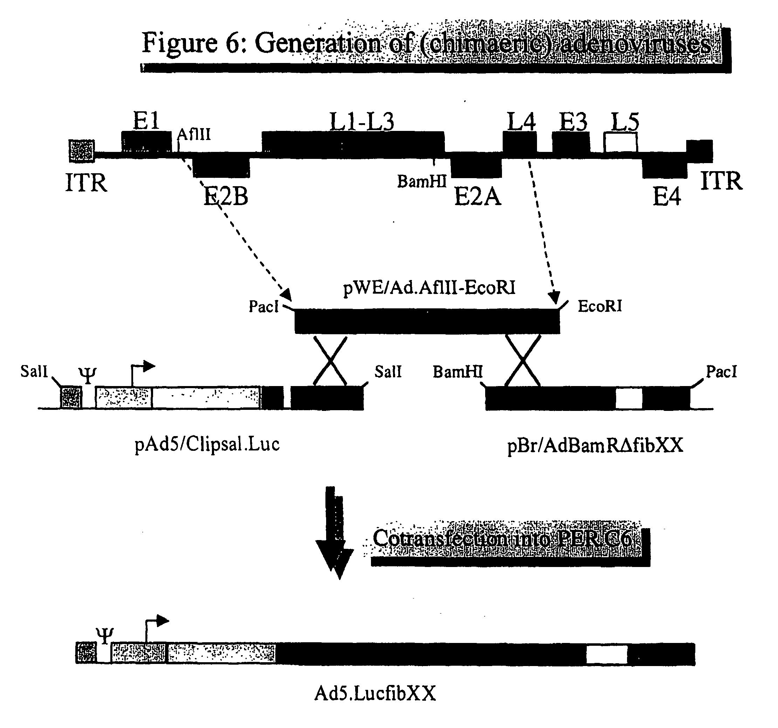

To enable efficient generation of chimaeric viruses an AvrII fragment from the pBr/AdBamRFib16, pBr/AdBamRFib28, pBr/AdBamRFib40-L constructs was subcloned into the vector pBr/Ad.Bam-rITR.pac#8 (ECACC deposit #P97082121) replacing the corresponding sequences in this vector. pBr/Ad.Bam-rITR.pac#8 has the same adenoviral insert as pBr/Ad.Bam-rITR but has a PacI site near the rITR that enables the ITR to be separated from the vector sequences. The construct pWE/Ad.AflII-Eco was generated as follows. PWE.pac was digested with ClaI and the 5 prime protruding ends were filled in with Klenow enzyme. The DNA was then digested with PacI and isolate from agarose gel. PWE/AflIIrITR was digested with EcoRI and after treatment with Klenow enzyme digested with PacI. The large 24 kb. fragment containing the adenoviral sequences was isolated from agarose gel and ligated to the ClaI digested and blunted pWE.Pac vector. Use was made of the ligation express kit from Clontech. After transformation of XL10-gold cells from Stratagene, clones were identified that contained the expected construct. PWE/Ad.AlfII-Eco contains Ad5 sequences from basepairs 3534-27336. Three constructs, pClipsal-Luc (Figure 5) digested with SalI, pWE/Ad.AflII-Eco digested with PacI and EcoRI and pBr/AdBamR.pac/fibXX digested with BamHI and PacI were transfected into adenovirus producer cells (PER.C6, Fallaux *et al,* 1998). Figure 6 schematically depicts the method and fragments used to generate the chimaeric viruses. Only pBr/Ad.BamRfib12 was used without subcloning in the PacI containing vector and therefor was not liberated from vector sequences using PacI but was digested with ClaI which leaves approximately 160 bp of vector sequences attached to the right ITR. Furthermore, the pBr/Ad.BamRfib12 and pBr/Ad.BamRfib28 contain an internal BamHI site in the fiber sequences and were therefor digested with SalI which cuts in the vector sequences flanking the BamHI site. For transfection, 2 µg of pCLIPsal-Luc, and 4 µg of both pWE/Ad.AflII-Eco and pBr/AdBamR.pac/fibXX were diluted in serum free DMEM to 100 µl total volume. To this DNA suspension 100 µl 2.5x diluted lipofectamine (Gibco) in serum-free medium was added. After 30 minutes at room temperature the DNA-lipofectamine complex solution was added to 2.5 ml of serum-free DMEM which was subsequently added to a T25 cm² tissue culture flask. This flask contained PER.C6 cells that were seeded 24-hours prior to transfection at a density of 1x10⁶ cells/flask. Two hours later, the DNA-lipofectamine complex containing medium was diluted once by the addition of 2.5 ml DMEM supplemented with 20% fetal calf serum. Again 24 hours later the medium was replaced by fresh DMEM supplemented with 10% fetal calf serum. Cells were cultured for 6-8 days, subsequently harvested, and freeze/thawed 3 times. Cellular debris was removed by centrifugation for 5 minutes at 3000 rpm room temperature. Of the supernatant (12.5 ml) 3-5 ml was used to infect again PER.C6 cells (T80 cm² tissue culture flasks). This re-infection results in full cytopathogenic effect (CPE) after 5-6 days after which the adenovirus is harvested as described above.

### Production of fiber chimeric adenovirus

10 ml of the above described crude lysate was used to inoculate a 1 liter fermentor which contained 1 - 1.5 x 10⁶ PER.C6 cells/ml growing in suspension. Three days after inoculation, the cells were harvested and pelleted by centrifuging for 10 min at 1750 rpm at room temperature. The chimeric adenovirus present in the pelleted cells was subsequently extracted and purified using the following downstream processing protocol. The pellet was dissolved in 50 ml 10 mM NaPO₄⁻ and frozen at -20°C. After thawing at 37°C, 5.6 ml deoxycholate (5% w/v) was added after which the solution was homogenated. The solution was subsequently incubated for 15 minutes at 37°C to completely crack the cells. After homogenizing the solution, 1875 µl (1M) MgCl₂⁻ was added and 5 ml 100% glycerol. After the addition of 375 µl DNase (10 mg/ ml) the solution was incubated for 30 minutes at 37°C. Cell debris was removed by centrifugation at 1880xg for 30 minutes at room temperature without the brake on. The supernatant was subsequently purified from proteins by loading on 10 ml of freon. Upon centrifugation for 15 minutes at 2000 rpm without brake at room temperature three bands are visible of which the upper band represents the adenovirus. This band was isolated by pipetting after which it was loaded on a Tris/HCl (1M) buffered cesiumchloride blockgradient (range: 1.2 to 1.4 gr./ml). Upon centrifugation at 21000 rpm for 2.5 hours at 10°C the virus was purified from remaining protein and cell debris since the virus, in contrast to the other components, does not migrate into the 1.4 gr./ ml cesiumchloride solution. The virus band is isolated after which a second purification using a Tris/ HCl (1M) buffered continues gradient of 1.33 gr. /ml of cesiumchloride is performed. After virus loading on top of this gradient the virus is centrifuged for 17 hours at 55000 rpm at 10°C. Subsequently the virus band is isolated and after the addition of 30 µl of sucrose (50 w/v) excess cesiumchloride is removed by three rounds of dialysis, each round comprising 1 hour. For dialysis the virus is transferred to dialysis slides (Slide-a-lizer, cut off 10000 kDa, Pierce, USA). The buffers used for dialysis are PBS which are supplemented with an increasing concentration of sucrose (round 1 to 3: 30 ml, 60 ml, and 150 ml sucrose (50% w/v)/ 1.5 liter PBS, all supplemented with 7.5 ml 2% (w/v) CaMgCl₂). After dialysis, the virus is removed from the slide-a-lizer after which it is aliquoted in portions of 25 and 100 µl upon which the virus is stored at -85°C. To determine the number of virusparticles per ml, 50 µl of the virus batch is run on an high pressure liquid chromatograph (HPLC) as described by Shamram et al (1997). The virus titers were found to be in the same range as the Ad5.Luc virus batch (Ad5.Luc: 2.2 x 10¹¹vp/ ml; Ad5.LucFib12: 1.3 x 10¹¹vp/ ml; Ad5.LucFib16: 3.1 x 10¹² vp/ ml; Ad5.LucFib28: 5.4 x 10¹⁰ vp/ml; Ad5.LucFib40-L: 1.6 x 10¹² vp/ ml).

### Example 2: biodistribution of chimeric viruses after intravenous tail vein injection of rats.

To investigate the biodistribution of the chimeric adenoviruses carrying fiber 12, 16, 28, or 40-2, 1x10¹⁰ particles of each of the generated virusbatches was diluted in 1 ml PBS after which the virus was injected in the tail vein of adult male Wag/Rij rats (3 rats/ virus). As a control, Ad5 carrying the luciferase transgene was used. Forty-eight hours after the administration of the virus, the rats were sacrificed after which the liver, spleen, lung, kidney, heart, and brain were dissected. These organs were subsequently mixed with 1 ml of lysis buffer (1% Triton X-100/ PBS) and minced for 30 seconds to obtain a protein lysate. The protein lysate was subsequently tested for the presence of transgene expression (luciferase activity) and the protein concentration was determined to express the luciferase activity per µg of protein. The results, Shown in Table II, demonstrate that in contrast to the Adenovirus serotype 5 control, none of the fiber chimeras are targeted specifically to the liver or to the spleen. This experiment shows that it is possible to circumvent the uptake of adenoviruses by the liver by making use of fibers of other serotypes. It also shows that the uptake by the liver is not correlated with the length of the fiber shaft, or determined solely by the ability of fiber knob to bind to CAR. The fibers used have different shaft lengths and, except for fiber 16, are derived from subgroups known to have a fiber that can bind CAR (Roelvink et al 1998).

### Example 3: Chimeric viruses display differences in endothelial and smooth muscle cell transduction

### A) Infection of Human endothelial cells

Human endothelial cells (HUVEC) were isolated, cultured and characterized as described previously (Jaffe et al 1973; Wijnberg et al 1997). Briefly, cells were cultured on gelatin-coated dishes in M199 supplemented with 20 mM HEPES, pH 7.3 (Flow Labs., Irvine, Scotland), 10% (v/v) human serum (local blood bank), 10% (v/v) heat-inactivated newborn calf serum (NBCS) (GIBCO BRL, Gaithersburg, MD), 150 µg/ ml crude endothelial cell growth factor, 5 U/ ml heparin (Leo Pharmaceutics Products, Weesp, The Netherlands), penicillin (100 IU/ ml)/streptomycin (100 µg/ ml) (Boehringer Mannheim, Mannheim, FRG) at 37°C under 5% (v/v) CO₂/ 95% (v/v) air atmosphere. Cells used for experiments were between passage 1-3. In a first set of experiments 40000 HUVEC cells (a pool from 4 different individuals) were seeded in each well of 24-wells plates in a total volume of 200 µl. Twenty-four hours after seeding, the cells were washed with PBS after which 200 µl of DMEM supplemented with 2% FCS was added to the cells. This medium contained various amounts of virus (MOI = 50, 250, 1000, 2500, 5000, and 10000). The viruses used were besides the control Ad5, the fiber chimeras 12, 16, 28, and 40-L (each infection in triplicate). Two hours after addition of the virus the medium was replaced by normal medium. Again forty-eight hours later cells were washed and lysed by the addition of 100 µl lysisbuffer. In figure 7a, results are shown on the transgene expression per microgram total protein after infection of HUVEC cells. These results show that fiber chimeras 12 and 28 are unable to infect HUVEC cells, that 40-L infects HUVECs with similar efficiency as the control Ad5 virus, and that fiber chimera 16 infects HUVECs significantly better. In a next set of experiments (n = 8) the fiber 16 chimera was compared with the Ad5.Luc vector on HUVEC for luciferase activity after transduction with 2500 virus particles per cell of each virus. These experiments demonstrated that fiber 16 yields, on average, 8.1 fold increased luciferase activity (SD ± 4.6) as compared with Ad5. In a next experiment, an equal number of virus particles was added to wells of 24-well plates that contained different HUVEC cell concentrations. This experiment was performed since it is known that HUVECs are less efficiently infected with adenovirus serotype 5 when these cells reach confluency. For this purpose, HUVECs were seeded at 22500, 45000, 90000, and 135000 cells per well of 24-well plates (in triplicate). Twenty-four hours later these cells were infected as described above with medium containing 4.5 x 10⁸ virusparticles. The viruses used were, besides the control adenovirus serotype 5, the chimera fiber 16. The result of the transgene expression (RLU) per microgram protein determined 48 hours after infection (see figure 7b) shows that the fiber 16 chimeric adenovirus is also better suited to infect HUVEC cells even when these cells are 100% confluent which better mimics an *in vivo* situation. Since the Luciferase markergene does not provide information concerning the number of cells infected another experiment was performed with adenovirus serotype 5 and the fiber 16 chimera, both carrying a green fluorescent protein (GFP) as a marker gene. This protein expression can be detected using a flow cytometer which renders information about the percentage of cells transduced as well as fluorescence per cell. In this experiment cells were seeded at a concentration of 40000 cells per well and were exposed to virus for 2 hours. The virus used was Ad5.GFP (8.4 x 10¹¹ vp/ ml) and Ad5.Fib16.GFP (5.1 x 10¹¹ vp/ ml). Cells were exposed to a virus concentration of 500 virus particles per cell. Flow cytometric analysis, 48 hours after virus exposure demonstrated that the fiber 16 virus gives higher transgene expression levels per cell since the median fluorescence, a parameter identifying the amount of GFP expression per cell, is higher with fiber 16 as compared to Ad5 (Figure 7c). These results are thus consistent and demonstrate that the fiber 16 chimeric virus is better suited to infect human primary endothelial cells as compared to Ad5.

### B) Infection of human smooth muscle cells

Smooth muscle cells were isolated after isolation of EC (Weinberg et al 1997). The veins were incubated with medium (DMEM) supplemented with penicillin/ streptomycin) containing 0.075% (w/v) collagenase (Worthington Biochemical Corp., Freehold, NJ, USA). After 45 minutes the incubation medium containing detached cells was flushed from the veins. Cells were washed and cultured on gelatin coated dishes in culture medium supplemented with 10% fetal calf serum and 10% human serum at 37°C under 5% (v/v) CO₂/ 95% (v/v) air atmosphere. Cells used for experiments were between passage 3-6. We first tested the panel of chimeric fiber viruses versus the control adenovirus serotype 5 for the infection of human smooth muscle cells. For this purpose, 40000 human umbilical vein smooth muscle cells (HUVsmc) were seeded in wells of 24-well plates in a total volume of 200 µl. Twenty-four hours after seeding, the cells were washed with PBS after which 200 µl of DMEM supplemented with 2% FCS was added to the cells. This medium contained various amounts of virus (MOI = 50, 250, 1250, 2500, and 5000). The viruses used were besides the control Ad5 the fiber chimeras 12, 16, 28, and 40-L (each infection in triplicate). Two hours after addition of the virus the medium was replaced by normal medium. Again forty-eight hours later cells were washed and lysed by the addition of 100 µl lysisbuffer. In figure 8a, results are shown of the transgene expression per microgram total protein after infection of HUVsmc cells. These results show that fiber chimeras 12 and 28 are unable to infect HUVsmc cells, that 40-L infects HUVsmc with similar efficiency as the control Ad5 virus, and that fiber chimera 16 infects HUVsmc significantly better. In a next set of experiments, smooth muscle cells derived from saphenous vene, arteria Iliaca, left interior mammory artery (LIMA) and aorta were tested for infection with the fiber 16 chimera and Ad5 (both carrying luciferase as a marker gene). These experiments (n = 11) demonstrated that, on average, the fiber 16 chimera yielded 61.4 fold increased levels in luciferase activity (SD ± 54.8) as compared to Ad5. The high standard deviation (SD) is obtained due to the finding that the adenoviruses used vary in their efficiency of infection of SMC derived from different human vessels. In a next experiment, an equal number of virus particles was added to wells of 24-well plates that contained different HUVsmc cell concentrations confluency. For this purpose, HUVsmc were seeded at 10000, 20000, 40000, 60000, and 80000 cells per well of 24-well plates (in triplicate). Twenty-four hours later these cells were infected as described above with medium containing 2 x 10⁸ virusparticles. The viruses used were, besides the control adenovirus serotype 5, the chimera fiber 16. The result of the transgene expression (RLU) per microgram protein determined 48 hours after infection (see figure 8b) shows that the fiber 16 chimeric adenovirus is better suited to infect smooth muscle cells even when these cells are 100% confluent which better mimics an *in vivo* situation. To identify the number of SMCs transduced with the fiber 16 chimera and Ad5, we performed transduction experiments with Ad5.GFP and Ad5Fib16.GFP (identical batches as used for EC infections). Human umbilical vein SMC were seeded at a concentration of 60000 cells per well in 24-well plates and exposed for 2 hours to 500 or 5000 virus particles per cell of Ad5.GFP or Ad5Fib16.GFP. Forty-eight hours after exposure cells were harvested and analyzed using a flow cytometer. The results obtained show that the fiber 16 mutant yields approximately 10 fold higher transduction of SMC since the GFP expression measured after transduction with 5000 virus particles of Ad5.GFP is equal to GFP expression after transduction with 500 virus particles per cell of the fiber 16 chimera (Figure 8c).

### C) Subgroup B fiber mutants other than fiber 16

The shaft and knob of fiber 16 are derived from adenovirus serotype 16 which, as described earlier, belongs to subgroup B. Based on hemagglutination assays, DNA restriction patterns, and neutralization assays the subgroup B viruses have been further subdivided into subgroup B1 and B2 (Wadell et al 1984). Subgroup B1 members include serotypes 3, 7, 16, 21, and 51. Subgroup B2 members include 11, 14, 34, and 35. To test whether the increased infection of smooth muscle cells is a trade of all fibers derived from subgroup B or specific for one or more subgroup B fiber molecules, we compared fiber 16 and fiber 51 (both subgroup B1) with fiber 11 and fiber 35 (both subgroup B2). For this purpose HUVsmc were infected with increasing amounts of virus particles per cell (156, 312, 625, 1250, 2500, 5000). The fiber mutant all carry the Luciferase marker gene (Ad5Fib11.Luc: 1.1 x 10¹² vp/ml; Ad5Fib35Luc: 1.4 x 10¹² vp/ml ; Ad5Fib51Luc: 1.0 x 10¹² vp/ml). Based on the Luciferase activity measured and shown in Figure 8d, efficient infection of SMC is not a general trade of all subgroup B fiber molecules. Clearly fiber 16 an fiber 11 are better suited for infection of SMC than fiber 35 and fiber 51. Nevertheless, all subgroup B fiber mutants tested infect SMC better as compared to Ad5.

### D) Organ culture experiments

We next identified whether the observed difference in transduction of EC and SMC using the fiber 16 chimera or the Ad5 can also be demonstrated in organ culture experiments. Hereto, We focused on the following tissues: 1) Human Saphenous vein: the vein used in approximately 80% of all clinical vein grafting procedures 2) Human pericard/ epicard: for delivery of recombinant adenoviruses to the pericardial fluid which after infection of the percardial or epicardial cells produce the protein of interest from the transgene carried by the adenovirus. 3) Human coronary arteries: for percutaneous transluminal coronary angioplasty (PTCA) to prevent restenosis. Of the coronary arteries we focused on the Left artery descending (LAD) en right coronary artery (RCA).
Parts of a human saphenous vein left over after vein graft surgery were sliced into pieces of approximately 0.5 cm. These pieces (n=3) were subsequently cultured for 2 hours in 200 ml of 5 x10¹⁰ virus particles per ml. After two hours virus exposure the pieces were washed with PBS and cultured for another 48 hours at 37°C in a 10% CO2 incubator. The pieces were then washed fixated and stained for LacZ transgene expression. The viruses were Ad5.LacZ (2.2 x 10¹² vp/ ml), the fiber 16 chimera Ad5Fib16.LacZ (5.2 x 10¹¹ vp/ml), and A fiber 51 chimera: Ad5Fib51.LacZ (2.1 x 10¹² vp/ml). The pieces of saphenous vein were macroscopically photographed using a digital camera. Based on LacZ transgene expression obtained after 2 hours of virus exposure on saphenous vein slices, both the fiber 16 and the fiber 51 chimeric viruses give higher infection since much more blue staining is observed using these viruses as compared to Ad5.LacZ (Figure 8e). Identical experiments as described on saphenous vein were performed with human pericard and the human coronary arteries: RCA and LAD. Results of these experiments (Figures 8f-8g-8h respectively) together with the experiments performed on primary cells confirmed the superiority of the fiber 16 and 51 mutants as compared to Ad5 in infecting human cardiovascular tissues.

### E) CAR and integrin expression on human EC and SMC

From the above described results it is clear that the chimeric adenovirus with the shaft and knob from fiber 16 is well suited to infect endothelial cells and smooth muscle cells. Thus, by changing the fiber protein on Ad5 viruses we are able to increase infection of cells that are poorly infected by Ad5. The difference between Ad5 and Ad5Fib16, although significant on both cell types, is less striking on endothelial cells as compared to smooth muscle cells. This may reflect differences in receptor expression. For example, HUVsmc express significantly more αᵥβ5 integrins than HUVEC (see below). Alternatively, this difference may be due to differences in expression of the receptor of fiber 16. Ad5.LucFib16 infects umbilical vein smooth muscle cells approximately 8 fold better than umbilical vein endothelial cells whereas in case of Ad5.Luc viruses endothelial cells are better infected than smooth muscle cells. To test whether Ad5 infection correlated with receptor expression of these cells the presence of CAR and αᵥ-integrins was assayed on a flow cytometer. For this purpose 1x10⁵ HUVEC cells or HUVsmc were washed once with PBS/ 0.5% BSA after which the cells were pelleted by centrifugation for 5 minutes at 1750 rpm at room temperature. Subsequently, 10 µl of a 100 times diluted αᵥβ3 antibody (Mab 1961, Brunswick chemie, Amsterdam, The Netherlands), a 100 times diluted antibody αᵥβ5 (antibody (Mab 1976, Brunswick chemie, Amsterdam, The Netherlands), or 2000 times diluted CAR antibody was a kind gift of Dr. Bergelson, Harvard Medical School, Boston, USA (Hsu et al) was added to the cell pellet after which the cells were incubated for 30 minutes at 4°C in a dark environment. After this incubation, cells were washed twice with PBS/0.5% BSA and again pelleted by centrifugation for 5 minutes at 1750 rpm room temperature. To label the cells, 10 ml of rat anti mouse IgG1 labeled with phycoerythrine (PE) was added to the cell pellet upon which the cells were again incubated for 30 minutes at 4°C in a dark environment. Finally the cells were washed twice with PBS/0.5% BSA and analyzed on a flow cytometer. The results of these experiments are shown in table III. From the results it can be concluded that HUVsmc do not express detectable levels of CAR confirming that these cells are difficult to transduce with an adenovirus which enters the cells via the CAR receptor.

### F) Infection of human A549 cells

As a control for the experiments performed on endothelial cells and smooth muscle cells, A549 cells were infected to establish whether an equal amount of virus particles of the different chimeric adenoviruses show significant differences in transgene expression on cell lines that are easily infected by adenovirus. This is to investigate whether the observed differences in infection efficiency on endothelial and smooth muscle cells are cell type specific. For this purpose, 10⁵ A549 cells were seeded in 24-well plates in a volume of 200 µl. Two hours after seeding the medium was replaced by medium containing different amounts of particles of either fiber chimera 5, 12, 16, or 40-L (MOI = 0, 5, 10, 25, 100, 500). Twenty-four hours after the addition of virus, the cells were washed once with PBS after which the cells were lysed by the addition of 100 µl lysisbuffer to each well (1% Triton X-100 in PBS) after which transgene expression (Luciferase activity) and the protein concentration was determined. Subsequently, the luciferase activity per µg protein was calculated. The data, shown in table IV, demonstrate that Ad5.Luc viruses infect A549 cells most efficient while the infection efficiency of Ad5LucFib16 or Ad5LucFib40-L is a few times lower. This means that the efficient infection of endothelial cells and especially smooth muscle cells is due to differences in binding of the virus to these cells and not to the amount of virus or the quality of the viruses used.

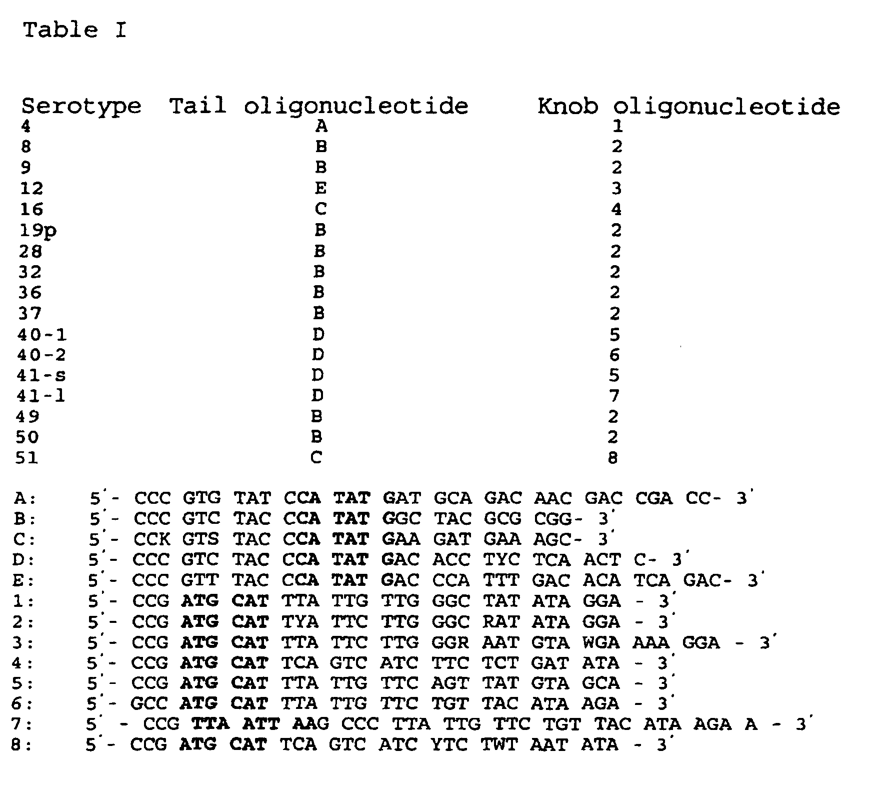

**Table II**

| **Organ** | **Control Ad5** | **Fib 12** | **Fib 16** | **Fib 28** | **Fib 40-L** |
|---|---|---|---|---|---|
| Liver | 740045 | 458 | 8844 | 419 | 2033 |
| Spleen | 105432 | 931 | 3442 | 592 | 16107 |
| Lung | 428 | 315 | 334 | 316 | 424 |
| Kidney | 254 | 142 | 190 | 209 | 224 |
| Heart | 474 | 473 | 276 | 304 | 302 |
| Brain | 291 | 318 | 294 | 323 | 257 |

**Table III**

| Cell line | αᵥβ3 | αᵥβ5 | CAR |
|---|---|---|---|
| HUVEC 70% | 98.3% | 18.9% | 18.1% |
| HUVEC 100% | 97.2% | 10.5% | 7.2% |
| HUVsmc 70% | 95.5% | 76.6% | 0.3% |
| HUVsmc 100% | 92.2% | 66.5% | 0.3% |
| PER.C6 | 7.8% | 16.8% | 99.6% |

**Table IV**

| MOI (VP/Cell) | Control Ad5 | Fiber 12 | Fiber 16 | Fiber 40-L |
|---|---|---|---|---|
| 0 | 0 | 0 | 0 | 0 |
| 5 | 1025 | 46 | 661 | 443 |
| 10 | 1982 | 183 | 1704 | 843 |
| 25 | 4840 | 200 | 3274 | 2614 |
| 100 | 21875 | 1216 | 13432 | 11907 |
| 500 | 203834 | 3296 | 93163 | 71433 |

### REFERENCES

Arnberg N., Mei Y. and Wadell G., 1997. Fiber genes of adenoviruses with tropism for the eye and the genital tract. Virology 227: 239-244.

Bergelson, J.M., Cunningham, J.A., Droguett, G., Kurt-Jones, E.A., Krithivas, A., Hong, J.S., Horwitz, M.S., Crowell, R.L. and Finberg, R.W. (1997) Isolation of a common receptor for coxsackie B virus and adenoviruses 2 and 5. Science 275: 1320-1323.

Bout A. (1997) Gene therapy, p. 167-182. In: D.J.A. Crommelin and R.D. Sindelar (ed.), Pharmaceutical Biotechnology, Harwood Academic Publishers.

Bout, A. (1996) Prospects for human gene therapy. Eur. J. Drug Met. and Pharma. 2, 175-179.

Blaese, M., Blankenstein, T., Brenner, M., Cohen-Hagenauer, O., Gansbacher, B., Russel, S., Sorrentino, B. and Velu, T. (1995) Cancer Gene Ther. 2: 291-297.

Brody, S.L. and Crystal, R.G. (1994) Adenovirus mediated in vivo gene transfer. Ann. N. Y. Acad. Sci. 716:90-101.

Carter, A.J., Laird, J.R., Farb, A., Kufs, W., Wortham, D.C. and Virmani, R. (1994) Morphologic characteristics of lesion formation and time course of smooth muscle cell proliferation in a porcine proliferative restenosis model. J. Am. Coll. Cardiol. 24: 1398-1405.

Chroboczek J., Ruigrok R.W.H., and Cusack S., 1995. Adenovirus fiber, p. 163-200. In: W. Doerfler and P. Bohm (ed.), The molecular repertoire of adenoviruses, I. Springer-Verlag, Berlin.

Defer C., Belin M., Caillet-Boudin M. and Boulanger P., 1990. Human adenovirus-host cell interactions; comparative study with members of subgroup B and C. Journal of Virology 64 (8): 3661-3673.

Fallaux, F.J, Bout, A, van der Velde, I et al. New helper cells and matched E1-deleted adenovirus vectors prevent generation of replication competent adenoviruses. Human Gene Therapy, 9 (1998), p1909-1917.

Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2: 140-144.

Gall J., Kass-Eisler A., Leinwand L. and Falck-Pedersen E. (1996) Adenovirus type 5 and 7 capsid chimera: fiber replacement alters receptor tropism without affecting primary immune neutralization epitopes. Journal of Virology 70 (4): 2116-2123.

Greber, U.F., Willets, M., Webster, P., and Helenius, A. (1993). Stepwise dismanteling of adenovirus 2 during entry into cells. Cell 75: 477-486.

Hynes, R.O. (1992) Integrins: versatility, modulation and signalling in cell adhesion. Cell 69: 11-25.

Herz, J. and Gerard, R.D. (1993) Adenovirus-mediated transfer of low density lipoprotein receptor gene acutely accelerates cholesterol clearence in normal mice. Proc. Natl. Acad. Sci. U.S.A. 96: 2812-2816.

Hierholzer, J.C. (1992) Adenovirus in the immunocompromised host. Clin. Microbiol Rev. 5, 262-274.

Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types 43-47). J. Infect. Dis. 158, 804-813.

Hong, S.S., Karayan, L., Tournier, J., Curiel, D.T. and Boulanger, P.A. (1997) Adenovirus type 5 fiber knob binds to MHC class I α2 domain at the surface of human epithelial and B lymphoblastoid cells. EMBO J. 16: 2294-2306.

Hsu, K.H., Lonberg-Holm, K., Alstein, B. and Crowell, R.L. (1988)
A monoclonal antibody specific for the cellular receptor for the group B coxsackieviruses. J. Virol 62(5): 1647-1652.

Huard, J., Lochmuller, H., Acsadi, G., Jani, A., Massie, B. and Karpati, G. (1995) The route of administration is a major determinant of the transduction efficiency of rat tissues by adenoviral recombinants. Gene Ther. 2: 107-115.

Ishibashi, M. and Yasue, H. (1984) The adenoviruses, H.S. Ginsberg, ed., Plenum Press, Londen, New York. Chapter 12, 497-561.

Jaffe, E.A., Nachman, R.L., Becker, C.G., Minick, C.R. (1973) Culture of endothelial cells derived from umbilical veins. Identification by morphologic and immunologic criteria. *J. Clin. Invest.* 52, 2745-2756.

Kass-Eisler, A., Falck-Pederson, E., Elfenbein, D.H., Alvira, M., Buttrick, P.M. and Leinwand, L.A. (1994) The impact of developmental stage, route of administration and the immune system on adenovirus-mediated gene transfer. Gene Ther. 1: 395-402.

Khoo, S.H., Bailey, A.S., De Jong, J.C., and Mandal, B.K. (1995). Adenovirus infections in human immunodeficiency virus-positive patients: Clinical features and molecular epidemiology. J. Infect. Dis 172, 629-637

Kidd, A.H., Chroboczek, J., Cusack, S., and Ruigrok, R.W. (1993) Adenovirus type 40 virions contain two distinct fibers. Virology 192, 73-84.

Krasnykh V.N., Mikheeva G.V., Douglas J.T. and Curiel D.T. (1996) Generation of recombinant adenovirus vectors with modified fibers for altering viral tropism. J. Virol. 70(10): 6839-6846.

Krasnykh V.N., Dmitriev I., Mikheeva G., Miller C.R., Belousova N. and Curiel D.T. (1998) Characterization of an adenovirus vector containing a heterologous peptide epitope in the HI loop of the fiber knob. J. Virol. 72(3): 1844-1852.

Law, L., Chillon, M., Bosch, A., Armentano, D., Welsh, M.J. and Davidson, B.L. (1998) Infection of primary CNS cells by different adenoviral serotypes: Searching for a more efficient vector. Abstract 1^{st} Annual Meeting American Society of Gene Therapy, Seattle, Washington.

Leppard, K.N. (1997) E4 gene function in adenovirus, adenovirus vector and adeno-associated virus infections. J. Gen. Virol. 78: 2131-2138.

Lloyd Jones, D.M. and Bloch, K.D. (1996) The vascular biology of nitric oxide and its role in atherogenesis. Annu. Rev. Med. 47: 365-375.

Morgan, C., Rozenkrantz, H.S., and Mednis, B. (1969) Structure and development of viruses as observed in the electron microscope.X. Entry and uncoating of adenovirus. J. Virol 4, 777-796.

Roelvink, P.W., Kovesdi, I. and Wickham, T.J. (1996) Comparative analysis of adenovirus fiber-cell interaction: Adenovirus type 2 (Ad2) and Ad9 utilize the same cellular fiber receptor but use different binding stratagies for attachemnt. J. Virol. 70: 7614-7621.

Roelvink, P.W., Lizonova, A., Lee, J.G.M., Li, Y., Bergelson, J.M., Finberg, R.W., Brough, D.E., Kovesdi, I. and Wickham, T.J. (1998) The coxsackie-adenovirus receptor protein can function as a cellular attachment protein for adenovirus serotypes from subgroups A, C, D, E, and F. J. Virol. 72: 7909-7915.

Rogers, B.E., Douglas J.T., Ahlem, C., Buchsbaum, D.J., Frincke, J. and Curiel, D.T. (1997) Use of a novel crosslinking method to modify adenovirus tropism. Gene Ther.4: 1387-1392.

Schulick, A.H., Vassalli, G., Dunn, P.F., Dong, G., Rade, J.J., Zamarron, C. and Dichek, D.A. (1997). Established immunity precludes adenovirus-mediated gene transfer in rat carotid arteries.

Schnurr, D and Dondero, M.E. (1993) Two new candidate adenovirus serotypes. Intervirol. 36, 79-83.

Schwartz, R.S., Edwards, W.D., Huber, K.C., Antoniudes, L.C. Bailey, K.R., Camrud, A.R., Jorgenson, M.A. and Holmes, D.R. Jr. (1993) Coronary restenosis: Prospects for solution and new perspectives from a porcine model. Mayo Clin. Proc. 68: 54-62.

Shi, Y., Pieniek, M., Fard, A., O'Brien, J., Mannion, J.D. and Zalewski, A. (1996) Adventitial remodelling after coronary arterial injury. Circulation 93: 340-348.

Shabram, P.W., Giroux, D.D., Goudreau, A.M., Gregory, R.J., Horn, M.T., Huyghe, B.G., Liu, X., Nunnally, M.H., Sugarman, B.J. and Sutjipto, S. (1997) Analytical anion-exchange HPLC of recombinant type-5 adenoviral particles. Hum. Gene Ther. 8(4): 453-465.

Signas, G., Akusjarvi, G., and Petterson, U. (1985). Adenovirus 3 fiberpolypeptide gene: Complications for the structure of the fiber protein. J. Virol. 53, 672-678.

Stevenson S.C., Rollence M., White B., Weaver L. and McClelland A., (1995) Human adenovirus serotypes 3 and 5 bind to two different cellular receptors via the fiber head domain. J. Virol 69(5): 2850-2857.

Stevenson S.C., Rollence M., Marshall-Neff J. and McClelland A. (1997) Selective targeting of human cells by a chimaeric adenovirus vector containing a modified fiber protein. J. Virology 71(6): 4782-4790.

Stouten, P.W.F., Sander, C., Ruigrok, R.W.H., and Cusack, S. (1992) New triple helical model for the shaft of the adenovirus fiber. J. Mol. Biol. 226, 1073-1084.

Svensson, V. and Persson, R. (1984). Entry of adenovirus 2 into Hela cells. J. Virol. 51, 687-694.

Van der Vliet, P.C. (1995) Adenovirus DNA replication In: W. Doerfler and P. Bohm (eds.), The molecular repertoire of adenoviruses II. Springer-Verlag, Berlin.

Varga, M.J., Weibull, C., and Everitt, E. (1991). Infectious entry pathway of adenovirus type 2. J. Virol 65, 6061-6070.

Varenne, O., Pislaru, S., Gillijns, H., Van Pelt, N., Gerard, R.D., Zoldhelyi, P., Van de Werf, F., Collen, D. and Janssens, S.P. (1998) Local adenovirus-mediated transfer of human endothelial nitric oxide synthetase reduces luminal narrowing after coronary angioplasty in pigs. Circulation 98: 919-926.

Wadell G (1984) Molecular Epidemiology of human adenoviruses *Curr. Top. Microbiol.Immunol.* 110, 191-220.

Wickham T.J., Carrion M.E. and Kovesdi I., 1995. Targeting of adenovirus penton base to new receptors through replacement of its RGD motif with other receptor-specific peptide motifs. Gene Therapy **2**: 750-756.

Wickham T.J., Segal, D.M., Roelvink, P.W., Carrion M.E., Lizonova, A., Lee, G-M., and Kovesdi, I. (1996) Targeted adenovirus gene transfer to endothelial and smooth muscle cells by using bispecific antibodies. J. Virol. 70 (10), 6831-6838.

Wickham, T.J., Mathias, P., Cherish, D.A., and Nemerow, G.R. (1993) Integrins avb3 and avb5 promote adenovirus internalization but not virus attachment. Cell 73, 309-319.

Wijnberg, M.J., Quax, P.H.A., Nieuwenbroek, N.M.E., Verheijen, J.H. (1997). The migration of human smooth muscle cells in vitro is mediated by plasminogen activation and can be inhibited by al- pha(2)- macro globulin receptor associated protein. *Thromb. and Haemostas.* 78, 880-886.

Wold, W.S., Tollefson, A.E. and Hermiston, T.W. (1995) E3 transcription unit of adenovirus. In: W. Doerfler and P. Bohm (eds.), The molecular repertoire of adenoviruses I. Springer-Verlag, Berlin.

Zabner, J., Armentano, D., Chillon, M., Wadsworth, S.C. and Welsh, M.J. (1998) Type 17 fiber enhances gene transfer Abstract 1^{st} Annual Meeting American Society of Gene Therapy, Seattle, Washington.

## Claims

1. A recombinant adenoviral vector of a serotype from subgroup C, having been provided with at least a tissue tropism for smooth muscle cells and/or endothelial cells and having been deprived of at least a tissue tropism for liver cells, wherein said adenoviral vector comprises a capsid comprising a tropism determining fragment of a fiber protein of an adenovirus of a serotype from subgroup B, and wherein said adenovirus serotype from subgroup B is selected from the group consisting of: adenovirus 11, adenovirus 16, adenovirus 35 and adenovirus 51.

2. A recombinant adenoviral vector according to claim 1, further comprising an adenoviral nucleic acid from an adenovirus of subgroup C, said nucleic acid comprising a sequence encoding a fiber protein, said fiber protein comprising at least a tissue tropism determining fragment of a fiber protein of an adenovirus of a serotype from subgroup B, and wherein said adenovirus serotype from subgroup B is selected from the group consisting of: adenovirus 11, adenovirus 16, adenovirus 35 and adenovirus 51.

3. A recombinant adenoviral vector according to claim 1 or 2, wherein said serotype from subgroup C is adenovirus 5.

4. A recombinant adenoviral vector according to claim 3, wherein said adenoviral nucleic acid is modified such that the capacity of said adenoviral nucleic acid to replicate in a target cell has been reduced or disabled.

5. A recombinant adenoviral vector according to claims 3 or 4, further comprising at least one non-adenovirus nucleic acid.

6. A recombinant adenoviral vector according to claim 5, wherein at least one of said non-adenovirus nucleic acids is a gene selected from the group of genes encoding: an apolipoprotein, a nitric oxide synthase, a herpes simplex virus thymidine kinase, an interleukin-3, an interleukin-1alpha, an (anti)angiogenesis protein such as angiostatin, an anti-proliferation protein, a smooth muscle cell anti-migration protein, a vascular endothelial growth factor (VGEF), a basic fibroblast growth factor, a hypoxia inducible factor 1alpha (HIF-1alpha) or a PAI-1.

7. A recombinant adenoviral vector according to any one of claims 1 to 6 for use as a pharmaceutical.

8. Construct pBr/Ad.BamRΔFib, comprising adenovirus 5 sequences 21562-31094 and 32794-35938, wherein said construct lacks adenovirus 5 nucleotides 31095-32793 comprising the nucleic acid encoding at least the tissue tropism determining part of the fiber protein and further comprising a nucleic acid located between position 31094 and 32794 of adenovirus 5, said nucleic acid encoding at least a tissue tropism-determining fragment of the fiber protein of adenovirus 16, 11, 35, or 51.

9. Construct pBr/Ad BamR.pac/fib16, comprising adenovirus 5 sequences 21562-31094 and 32794-35938, comprising a nucleic acid located between position 31094 and 32794 of adenovirus 5, said nucleic acid encoding at least a tissue tropism-determining fragment of the fiber protein of adenovirus 16, and further comprising a unique PacI restriction site in the proximity of the adenovirus 5 right Inverted Terminal Repeat, in the non-adenoviral sequence backbone of said construct.

10. The use of a construct according to anyone of claims 8 to 9 for the generation of a recombinant adenoviral vector according to any one of claims 3 to 6.

## Patentansprüche

1. Rekombinanter adenoviraler Vektor eines Serotyps aus der Untergruppe C, der mit wenigstens einem Gewebetropismus für Zellen der glatten Muskulatur und/oder Endothelzellen ausgestattet ist und wenigstens von einem Gewebetropismus für Leberzellen befreit ist, wobei der adenovirale Vektor ein Kapsid umfasst, das ein tropismusbestimmendes Fragment eines Faserproteins eines Adenovirus eines Serotyps aus der Untergruppe B umfasst, und wobei der Adenovirus-Serotyp aus der Untergruppe B aus der Gruppe ausgewählt ist, die aus Adenovirus 11, Adenovirus 16, Adenovirus 35 und Adenovirus 51 besteht.

2. Rekombinanter adenoviraler Vektor gemäß Anspruch 1, der weiterhin eine adenovirale Nucleinsäure von einem Adenovirus der Untergruppe C umfasst, wobei die Nucleinsäure eine Sequenz umfasst, die ein Faserprotein codiert, wobei das Faserprotein wenigstens ein gewebetropismusbestimmendes Fragment eines Faserproteins eines Adenovirus eines Serotyps aus der Untergruppe B umfasst und wobei der Adenovirus-Serotyp aus der Untergruppe B aus der Gruppe ausgewählt ist, die aus Adenovirus 11, Adenovirus 16, Adenovirus 35 und Adenovirus 51 besteht.

3. Rekombinanter adenoviraler Vektor gemäß Anspruch 1 oder 2, wobei es sich bei dem Serotyp aus der Untergruppe C um Adenovirus 5 handelt.

4. Rekombinanter adenoviraler Vektor gemäß Anspruch 3, wobei die adenovirale Nucleinsäure so modifiziert ist, dass die Kapazität der adenoviralen Nucleinsäure zur Replikation in einer Zielzelle reduziert oder beseitigt ist,

5. Rekombinanter adenoviraler Vektor gemäß Anspruch 3 oder 4, der weiterhin wenigstens eine Nicht-Adenovirus-Nucleinsäure umfasst.

6. Rekombinanter adenoviraler Vektor gemäß Anspruch 5, wobei wenigstens eine der Nicht-Adenovirus-Nucleinsäuren ein Gen ist, das aus der Gruppe von Genen ausgewählt ist, die Folgendes codieren: ein Apolipoprotein, eine NO-Synthase, eine Herpes-simplex-Virus-Thymidin-Kinase, ein Interleukin-3, ein Interleukin-1α, ein (Anti)angiogeneseprotein, wie Angiostatin, ein Antiproliferationsprotein, ein Antimigrationsprotein von Zellen der glatten Muskulatur, einen Gefäßendothel-Wachstumsfaktor (VGEF), einen basischen Fibroblasten-Wachstumsfaktor, einen Hypoxie-induzierbaren Faktor 1α (HIF-1α) oder ein PAI-1.

7. Rekombinanter adenoviraler Vektor gemäß einem der Ansprüche 1 bis 6 zur Verwendung als Pharmakon.

8. Konstrukt pBr/Ad.BamRΔFib, das die Adenovirus-5-Sequenzen 21562-31094 und 32794-35938 umfasst, wobei dem Konstrukt die Adenovirus-5-Nucleotide 31095-32793 fehlen, die die Nucleinsäure umfassen, welche wenigstens den gewebetropismusbestimmenden Teil des Faserproteins codiert, und das weiterhin eine Nucleinsäure umfasst, die sich zwischen Position 31094 und 32794 des Adenovirus 5 befindet, wobei die Nucleinsäure wenigstens ein gewebetropismusbestimmendes Fragment des Faserproteins von Adenovirus 16, 11, 35 oder 51 codiert.

9. Konstrukt pBr/Ad.BamR.pac/fib16, das die Adenovirus-5-Sequenzen 21562-31094 und 32794-35938 umfasst und das eine Nucleinsäure umfasst, die sich zwischen Position 31094 und 32794 des Adenovirus 5 befindet, wobei die Nucleinsäure wenigstens ein gewebetropismusbestimmendes Fragment des Faserproteins von Adenovirus 16 codiert, und das weiterhin eine einzigartige PacI-Restriktionsstelle in der Nähe des rechten Inverted Terminal Repeat von Adenovirus 5 in dem nichtadenoviralen Sequenzgerüst des Konstrukts umfasst.

10. Verwendung eines Konstrukts gemäß einem der Ansprüche 8 bis 9 für die Herstellung eines rekombinanten adenoviralen Vektors gemäß einem der Ansprüche 3 bis 6.

## Revendications

1. Vecteur adénoviral recombinant d'un sérotype de sous-groupe C, ayant été muni d'au moins un tropisme tissulaire pour des cellules des muscles lisses et/ou des cellules endothéliales et ayant été privé d'au moins un tropisme cellulaire pour des cellules du foie, dans lequel ledit vecteur adénoviral comprend une capside comprenant un fragment d'une protéine fibre déterminant un tropisme d'un adénovirus d'un sérotype de sous-groupe B, et dans lequel ledit sérotype d'adénovirus de sous-groupe B est choisi parmi le groupe constitué de : l'adénovirus 11, l'adénovirus 16, l'adénovirus 35 et l'adénovirus 51.

2. Vecteur adénoviral recombinant selon la revendication 1, comprenant de plus un acide nucléique adénoviral d'un adénovirus de sous-groupe C, ledit acide nucléique comprenant une séquence codant une protéine fibre, ladite protéine fibre comprenant au moins un fragment d'une protéine fibre déterminant le tropisme tissulaire d'un adénovirus d'un sérotype de sous-groupe B, et dans lequel ledit sérotype d'adénovirus de sous-groupe B est choisi parmi le groupe constitué de : l'adénovirus 11, l'adénovirus 16, l'adénovirus 35 et l'adénovirus 51.

3. Vecteur adénoviral recombinant selon la revendication 1 ou 2, dans lequel ledit sérotype de sous-groupe C est l'adénovirus 5.

4. Vecteur adénoviral recombinant selon la revendication 3, dans lequel ledit acide nucléique adénoviral est modifié de telle sorte que la capacité dudit acide nucléique adénoviral à se répliquer dans une cellule cible a été réduite ou supprimée.

5. Vecteur adénoviral recombinant selon la revendication 3 ou 4, comprenant de plus au moins un acide nucléique non-adénoviral.

6. Vecteur adénoviral recombinant selon la revendication 5, dans lequel au moins l'un desdits acides nucléiques non-adénoviraux est un gène choisi parmi le groupe de gènes codant : une apolipoprotéine, une synthase d'oxyde azotique, une thymidine kinase du virus de l'herpès simplex, une interleukine-3, une interleukine-1alpha, une protéine (anti)angiogenèse telle qu'une angiostatine, une protéine anti-prolifération, une protéine anti-migration des cellules des muscles lisses, un facteur de croissance endothélial vasculaire (VGEF), un facteur de croissance des fibroblastes basiques, un facteur inductible d'hypoxie 1alpha (HIF-1alpha) ou un PAI-1.

7. Vecteur adénoviral recombinant selon une quelconque des revendications 1 à 6 pour une utilisation en tant que produit pharmaceutique.

8. Produit de synthèse pBr/Ad.BamRΔFib, comprenant des séquences 21562-31094 et 32794-35938 de l'adénovirus 5, dans lequel ledit produit de synthèse est dépourvu des nucléotides 31095-32793 de l'adénovirus 5 comprenant l'acide nucléique codant au moins la partie de la protéine fibre déterminant le tropisme tissulaire et comprenant de plus un acide nucléique situé entre la position 31094 et 32794 de l'adénovirus 5, ledit acide nucléique codant au moins un fragment de la protéine fibre déterminant le tropisme tissulaire de l'adénovirus 16, 11, 35 ou 51.

9. Produit de synthèse pBr/Ad BamR.pac/fib.16, comprenant les séquences 21562-31094 et 32794-35938 de l'adénovirus 5, comprenant un acide nucléique situé entre la position 31094 et 32794 de l'adénovirus 5, ledit acide nucléique codant au moins un fragment de la protéine fibre déterminant le tropisme tissulaire de l'adénovirus 16, et comprenant de plus un site de restriction unique PacI à proximité de la séquence terminale inverse droite de l'adénovirus 5, dans l'ossature de la séquence non-adénovirale dudit produit de synthèse.

10. Utilisation d'un produit de synthèse selon une quelconque des revendications 8 à 9, pour la réalisation d'un vecteur adénoviral recombinant selon une quelconque des revendications 3 à 6.
